# EUROPEAN PATENT APPLICATION

(11) **EP 4 130 274 A1**
(43) Date of publication of application: **08.02.2023**
(21) Application number: 21781411.0
(22) Date of filing: 31.03.2021
(51) Int. Cl.: C12N 15/52, C07K 14/415, C12N 1/15, C12N 1/19, C12N 1/21, C12N 5/10, C12N 15/29

(54) **METHOD FOR EDITING TARGET RNA**

(30) Priority: 31.03.2020 JP 2020065065
(71) Applicant: Editforce, Inc., Fukuoka-shi, Fukuoka, 810-0001 (JP)
(72) Inventor: ICHINOSE, Mizuho, Nagoya-shi, Aichi 464-8601 (JP); GUTMANN, Bernard, Fukuoka-shi, Fukuoka 810-0001 (JP); YAGI, Yusuke, Fukuoka-shi, Fukuoka 810-0001 (JP); AKAIWA, Yumi, Fukuoka-shi, Fukuoka 810-0001 (JP); SHIMAJIRI, Yasuka, Fukuoka-shi, Fukuoka 810-0001 (JP); NAKAMURA, Takahiro, Fukuoka-shi, Fukuoka 819-0395 (JP)
(74) Representative: Hoffmann Eitle
(86) International application number: PCT/JP2021/014096
(87) International publication number: WO 2021/201198

(57) **Abstract**

A method for converting an editing target C contained in a target RNA to U or an editing target U contained in a target RNA to C is provided. A method for editing a target RNA, which comprises applying an artificial DYW protein containing a DYW domain consisting of any one of the polypeptides a, b, c, and be mentioned below to the target RNA: a. a polypeptide having xₐ₁PGxₐ₂SWIExₐ₃-xₐ₁₆HP ... HxₐₐE ... Cxₐ₁₇xₐ₁₈CH ... DYW, having a sequence identity of at least 40% to the sequence of SEQ ID NO: 1, and having a C-to-U/U-to-C editing activity, b. a polypeptide having x_{b1}PGx_{b2}SWWTDx_{b3}-x_{b16}HP ... Hx_{bb}E ... Cx_{b17}x_{b18}CH ... DYW, having a sequence identity of at least 40% to the sequence of SEQ ID NO: 2, and having a C-to-U/U-to-C editing activity, c. a polypeptide having KPAx_{c1}Ax_{c2}IEx_{c3} ... Hx_{cc}E ... Cx_{c4}x_{c5}CH ... x_{c6}x_{c7}x_{c8}, having a sequence identity of at least 40% to the sequence of SEQ ID NO: 3, and having a C-to-U/U-to-C editing activity, and be. a polypeptide having x_{b1}PGx_{b2}SWWTDx_{b3}-x_{b16}HP ... Hx_{cc}E ... Cx_{c4}x_{c5}CH ... Dx_{bc1}x_{bc2}, having a sequence identity of at least 40% to the sequence of SEQ ID NO: 90, and having a C-to-U/U-to-C editing activity (in the sequences, x represents an arbitrary amino acid, and ... represents an arbitrary polypeptide fragment).

## Description

The present invention relates to an RNA editing technique using a protein that can bind to a target RNA. The present invention is useful in a wide range of fields such as medical care (drug discovery support, treatment etc.), agriculture (agricultural, marine and livestock production and breeding, etc), and chemistry (biological material production).

### Background Art

It is known that RNA editing, in which specific bases in the genome are replaced at the RNA level, frequently occurs in plant mitochondria and chloroplasts, and the pentatricopeptide repeat (PPR) proteins, RNA binding proteins, are involved in this phenomenon.

PPR proteins are classified into two families, P and PLS, according to the structures of the PPR motifs that constitute the proteins (Non-patent document 1). P class PPR proteins consist of a simple repeat of the standard 35 amino acid PPR motif (P), whereas PLS proteins contain, in addition to P, 2 kinds of motifs called L and S, which are similar to P. The PPR array in PLS proteins (array of PPR motifs) is constituted by P-L-S repeating units, first of which, P1L1S1, consists of three PPR motifs, P1 (about 35 amino acids), L1 (about 35 amino acids), and S1 (about 31 amino acids), followed on the C-terminal side of P1L1S1 by another P-L-S repeating unit slightly different in sequences consisting of P2 (35 amino acids), L2 (36 amino acids), and S2 (32 amino acids) motifs. Further, in addition to those consisting of P-L-S repeating units, there are those in which SS (31 amino acids) is repeated. In addition, the most downstream P2L2S2 motif may be followed on the C-terminal side by two PPR-like motifs called E1 and E2, and a DYW domain with a 136 amino acids cytidine deaminase-like domain sequence (Non-patent document 2).

The interactions of PPR proteins with RNA are specified by the PPR codes, which specify the binding RNA bases by a combination of several amino acids in each PPR motif, and these amino acids bind to the corresponding nucleotides via hydrogen bonds (Patent documents 1 and 2, and Non-patent documents 3 to 8).

### Prior Art References

### Patent documents

Patent document 1: International Publication WO2013/058404
Patent document 2: International Publication WO2014/175284

### Non-patent documents

Non-patent document 1: Lurin, C., Andres, C., Aubourg, S., Bellaoui, M., Bitton, F., Bruyere, C., Caboche, M., Debast, C., Gualberto, J., Hoffmann, B., et al. (2004). Genome-wide analysis of Arabidopsis pentatricopeptide repeat proteins reveals their essential role in organelle biogenesis, Plant Cell 16:2089-2103
Non-patent document 2: Cheng, S., Gutmann, B., Zhong, X., Ye, Y., Fisher, M. F., Bai, F., Castleden, I., Song, Y, Song, B., Huang, J., et al. (2016) Redefining the structural motifs that determine RNA binding and RNA editing by pentatricopeptide repeat proteins in land plants, Plant J. 85:532-547
Non-patent document 3: Barkan, A., Rojas, M., Fujii, S., Yap, A., Chong, Y. S., Bond, C. S., and Small, 1. (2012) A combinatorial amino acid code for RNA recognition by pentatricopeptide repeat proteins, PLoS Genet. 8:e1002910
Non-patent document 4: Shen, C., Zhang, D., Guan, Z., Liu, Y, Yang, Z., Yang, Y, Wang, X., Wang, Q., Zhang, Q., Fan, S., et al. (2016) Structural basis for specific single-stranded RNA recognition by designer pentatricopeptide repeat proteins, Nat. Commun. 7:11285
Non-patent document 5: Yagi, Y, Hayashi, S., Kobayashi, K., Hirayama, T., and Nakamura, T. (2013) Elucidation of the RNA recognition code for pentatricopeptide repeat proteins involved in organelle RNA editing in plants, PLoS One 8:e57286 Non-patent document 6: Kobayashi, T., Yagi, Y, and Nakamura, T. (2019) Comprehensive Prediction of Target RNA Editing Sites for PLS-Class PPR Proteins in Arabidopsis thaliana, Plant Cell Physiol., 60:862-874
Non-patent document 7: Yan, J., Yao, Y, Hong, S., Yang, Y, Shen, C., Zhang, Q., Zhang, D., Zou, T., and Yin, P. (2019) Delineation of pentatricopeptide repeat codes for target RNA prediction, Nucleic Acids Res., 47:3728-3738
Non-patent document 8: Takenaka, M., Zehrmann, A., Brennicke, A., and Graichen, K. (2013). Improved computational target site prediction for pentatricopeptide repeat RNA editing factors, PLoS One 8:e65343
Non-patent document 9: Knie, N., Grewe, F., Fischer, S., and Knoop, V. (2016) Reverse U-to-C editing exceeds C-to-U RNA editing in some ferns - a monilophyte-wide comparison of chloroplast and mitochondrial RNA editing suggests independent evolution of the two processes in both organelles, BMC Evol. Biol. 16:134
Non-patent document 10: Gerke, P., Szovenyi, P., Neubauer, A., Lenz, H., Gutmann, B., McDowell, R., Small, I., Schallenberg-Rudinger, M., and Knoop, V. (2020) Towards a plant model for enigmatic U-to-C RNA editing: the organelle genomes, transcriptomes, editomes and candidate RNA editing factors in the hornwort Anthoceros agrestis, New Phytologist 225: 1974-1992
Non-patent document 11: Gutmann B., Royan S., Schallenberg-Rudinger M., Lenz H., Castleden I.R., McDowell R., Vacher M.A., Tonti-Filippini J., Bond C.S., Knoop V., and Small I.D. (2020) The Expansion and Diversification of Pentatricopeptide Repeat RNA-Editing Factors in Plants, Molecular Plant 13:215-230
Non-patent document 12: Oldenkott, B., Yang, Y, Lesch, E., Knoop, V., and Schallenberg-Rudinger, M. (2019) Plant-type pentatricopeptide repeat proteins with a DYW domain drive C-to-U RNA editing in Escherichia coli, Communications Biology 2:85

### Summary of the Invention

### Object to be achieved by the invention

C-to-U RNA editing, in which the RNA base cytidine is replaced by uridine, commonly occurs in land plant organelles. However, U-to-C RNA editing, in which uridine is replaced by cytidine, is also observed in hornworts and in some lycophytes and ferns (Non-patent document 9). Two different bioinformatics studies have found a unique DYW domain that differs in sequence from the standard DYW domain in plants showing U-to-C RNA editing (Non-patent documents 10 and 11).

Two kinds of DYW:PG proteins derived from *Physcomitrella patens* were reported to exhibit C-to-U RNA editing activity in *E. coli* (Non-patent document 12). The DYW domains of plant groups that diverged in an early stage in the evolution of land plants are roughly classified into two groups. The first is called DYW:PG/WW group, which includes the DYW:PG type, the standard DYW domain, and the DYW:WW type with the PG box to which one tryptophan (W) is added. The second group is called DYW:KP, in which the PG box and three amino acids sequence at the C-terminus of the DYW domain have sequences different from those of the PG/WW group, and such a domain presents only in plants showing U-to-C RNA editing. The technique to change one base in an arbitrary RNA sequence to a specific base is useful in gene therapies and genetic mutagenesis techniques for industrial applications. Although various RNA-binding molecules have been developed so far, there is no established method for designing molecules that convert cytidine (C) to uridine (U), or conversely, uridine (U) to cytidine (C) in an arbitrary target RNA by using a DYW domain and artificial RNA-binding protein.

### Means for achieving the object

In the present case, we show that a C-terminal DYW domain-containing portion of a PPR-DYW protein can be used as a modular editing domain for RNA editing. When each of the three kinds of DYW domains designed in this study was fused to PPR-P or PLS array, the DYW:PG and DYW:WW domains showed a C-to-U RNA editing activity, and DYW:KP showed a U-to-C RNA editing activity.

The present invention provides the followings.
[1] A method for editing a target RNA, the method comprising
   applying to the target RNA an artificial DYW protein containing a DYW domain consisting of any one of the polypeptides a, b, c, and bc mentioned below:
   a. a polypeptide having xₐ₁PGxₐ₂SWIExₐ₃-xₐ₁₆HP ... HxₐₐE ... Cxₐ₁₇Xa₁₈CH ... DYW, having a sequence identity of at least 40% to the sequence of SEQ ID NO: 1, and having a C-to-U/U-to-C editing activity,
   b. a polypeptide having x_{b1}PGx_{b2}SWWTDx_{b3}-x_{b16}HP ... Hx_{bb}E ... Cx_{b17}x_{b18}CH ... DYW, having a sequence identity of at least 40% to the sequence of SEQ ID NO: 2, and having a C-to-U/U-to-C editing activity,
   c. a polypeptide having KPAx_{c1}Ax_{c2}IEx_{c3} ... Hx_{cc}E ... Cx_{c4}x_{c5}CH ... x_{c6}x_{c7}x_{c8}, having a sequence identity of at least 40% to the sequence of SEQ ID NO: 3, and having a C-to-U/U-to-C editing activity, and
   bc. a polypeptide having x_{b1}PGx_{b2}SWWTDx_{b3}-x_{b16}HP ... Hx_{cc}E ... Cx_{c4}x_{c5}CH ... Dx_{bc1}x_{bc2}, having a sequence identity of at least 40% to the sequence of SEQ ID NO: 90, and having a C-to-U/U-to-C editing activity (in the sequences, x represents an arbitrary amino acid, and ... represents an arbitrary polypeptide fragment).
[2] A DYW domain consisting of any one of the polypeptides a, b, c, and bc mentioned below:
   a. a polypeptide having xₐ₁PGxₐ₂SWIExₐ₃-xₐ₁₆HP ... HxₐₐE ... Cxₐ₁₇xₐ₁₈CH ... DYW, having a sequence identity of at least 40% to the sequence of SEQ ID NO: 1, and having a C-to-U/U-to-C editing activity,
   b. a polypeptide having x_{b1}PGx_{b2}SWWTDx_{b3}-x_{b16}HP ... Hx_{bb}E ... Cx_{b17}x_{b18}CH ... DYW, having a sequence identity of at least 40% to the sequence of SEQ ID NO: 2, and having a C-to-U/U-to-C editing activity,
   c. a polypeptide having KPAx_{c1}Ax_{c2}IEx_{c3} ... Hx_{cc}E ... Cx_{c4}x_{c5}CH ... x_{c6}x_{c7}x_{c8}, having a sequence identity of at least 40% to the sequence of SEQ ID NO: 3, and having a C-to-U/U-to-C editing activity, and
   bc. a polypeptide having x_{b1}PGx_{b2}SWWTDx_{b3}-x_{b16}HP ... Hx_{cc}E ... Cx_{c4}x_{c5}CH ... Dx_{bc1}x_{bc2}, having a sequence identity of at least 40% to the sequence of SEQ ID NO: 90, and having a C-to-U/U-to-C editing activity.
[3] A DYW protein containing
   an RNA-binding domain that contains at least one PPR motif and can sequence-specifically bind to a target RNA, and
   the DYW domain according to 2.
[4] The DYW protein according to 3, wherein the RNA-binding domain is of the PLS type.
[5] A method for editing a target RNA, the method comprising:
   applying to the target RNA a DYW domain consisting of the polypeptide c or be mentioned below to convert an editing target U to C:
   c. a polypeptide having KPAx_{c1}Ax_{c2}IEx_{c3} ... Hx_{cc}E ... Cx_{c4}X_{c5}CH ... X_{c6}X_{c7}X_{c8}, having a sequence identity of at least 40% to the sequence of SEQ ID NO: 3, and having a C-to-U/U-to-C editing activity, and
   bc. a polypeptide having x_{b1}PGx_{b2}SWWTDx_{b3}-x_{b16}HP ... Hx_{cc}E ... Cx_{c4}x_{c5}CH ... Dx_{bc1}x_{bc2}, having a sequence identity of at least 40% to the sequence of SEQ ID NO: 90, and having a C-to-U/U-to-C editing activity.
[6] The method according to 5, wherein the DYW domain is fused to an RNA-binding domain that contains at least one PPR motif, and can sequence-specifically bind to a target RNA according to the rules of the PPR codes.
[7] A composition for editing RNA in an eukaryotic cell, the composition containing the DYW domain according to 2.
[8] A nucleic acid encoding the DYW domain according to 2, or the DYW protein according to 3 or 4.
[9] A vector containing the nucleic acid according to 8.
[10] A cell (except for human individual) containing the vector according to 9.

[1] A method for editing a target RNA, the method comprising
   applying to the target RNA an artificial DYW protein containing a DYW domain consisting of any one of the polypeptides a to c mentioned below:
   a. a polypeptide having xₐ₁PGxₐ₂SWIExₐ₃-xₐ₁₆HP ... HSE ... Cxₐ₁₇xₐ₁₈CH ... DYW, having a sequence identity of at least 40% to the sequence of SEQ ID NO: 1, and having a C-to-U/U-to-C editing activity,
   b. a polypeptide having x_{b1}PGx_{b2}SWWTDx_{b3}-x_{b16}HP ... HSE ... Cx_{b17}x_{b18}CH ... DYW, having a sequence identity of at least 40% to the sequence of SEQ ID NO: 2, and having a C-to-U/U-to-C editing activity, and
   c. a polypeptide having KPAx_{c1}Ax_{c2}IEx_{c3} ... HAE ... Cx_{c4}x_{c5}CH ... Dx_{c6}x_{c7}, having a sequence identity of at least 40% to the sequence of SEQ ID NO: 3, and having a C-to-U/U-to-C editing activity
      (in the sequences, x represents an arbitrary amino acid, and ... represents an arbitrary polypeptide fragment).
[2] A DYW domain consisting of any one of the polypeptides a to c mentioned below:
   a. a polypeptide having xₐ₁PGxₐ₂SWIExₐ₃-xₐ₁₆HP ... HSE ... Cxₐ₁₇xₐ₁₈CH ... DYW, having a sequence identity of at least 40% to the sequence of SEQ ID NO: 1, and having a C-to-U/U-to-C editing activity,
   b. a polypeptide having x_{b1}PGx_{b2}SWWTDx_{b3}-x_{b16}HP ... HSE ... Cx_{b17}x_{b18}CH ... DYW, having a sequence identity of at least 40% to the sequence of SEQ ID NO: 2, and having a C-to-U/U-to-C editing activity, and
   c. a polypeptide having KPAx_{c1}Ax_{c2}IEx_{c3} ... HAE ... Cx_{c4}x_{c5}CH ... Dx_{c6}x_{c7}, having a sequence identity of at least 40% to the sequence of SEQ ID NO: 3, and having a C-to-U/U-to-C editing activity.
[3] A DYW protein containing
   an RNA-binding domain that contains at least one PPR motif and can sequence-specifically bind to a target RNA, and
   the DYW domain according to 2.
[4] The DYW protein according to 3, wherein the RNA-binding domain is of the PLS type.
[5] A method for editing a target RNA, the method comprising:
   applying to the target RNA a DYW domain consisting of the polypeptide c mentioned below to convert an editing target U to C:
   c. a polypeptide having KPAx_{c1}Ax_{c2}IEx_{c3} ... HAE ... Cx_{c4}x_{c5}CH ... Dx_{c6}x_{c7}, having a sequence identity of at least 40% to the sequence of SEQ ID NO: 3, and having a C-to-U/U-to-C editing activity.
[6] The method according to 5, wherein the DYW domain is fused to an RNA-binding domain that contains at least one PPR motif, and can sequence-specifically bind to a target RNA according to the rules of the PPR codes.
[7] A composition for editing RNA in an eukaryotic cell, the composition containing the DYW domain according to 2.
[8] A nucleic acid encoding the DYW domain according to 2, or the DYW protein according to 3 or 4.
[9] A vector containing the nucleic acid according to 8.
[10] A cell (except for human individual) containing the vector according to 9.

### Effect of the Invention

The present invention enables conversion of an editing target C to U, or an editing target U to C in a target RNA.

### Brief Description of the Drawings

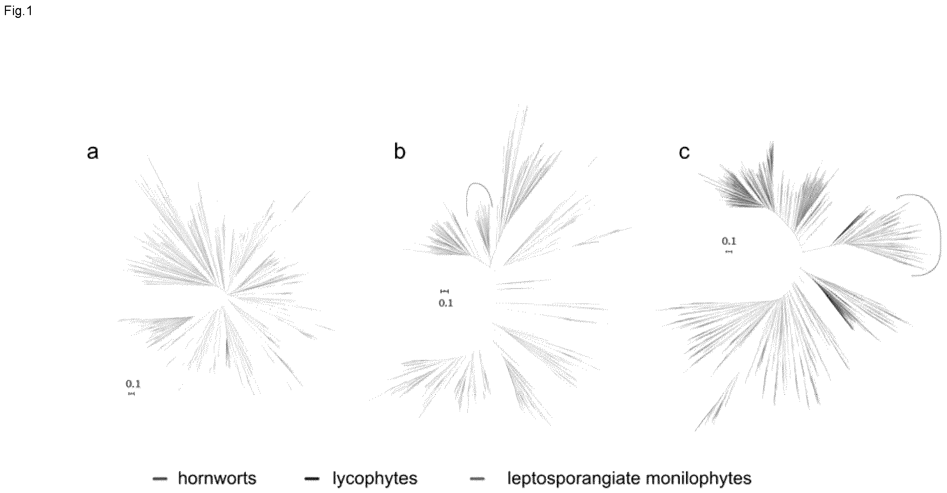

[Fig. 1] Approximate maximum likelihood phylogenetic trees of the C-terminal domain in the DYW proteins. Phylogenetic trees of (a) DYW:PG type, (b) DYW:WW type, and (c) DYW:KP type domains were generated by using FastTree. The DYW:PG domain was designed on the basis of a DYW:PG protein derived from lycophytes. The phylogenetic groups (clades) of the proteins selected to design the DYW:WW and DYW:KP domains are indicated with the black lines. The phylogenetic trees are visualized by using iTOL (Letunic, Land Bork, P. (2016) Nucleic Acids Res., 44 W242-245), with red indicating proteins derived from homworts, green indicating proteins derived from lycophytes, and blue indicating proteins derived from ferns.

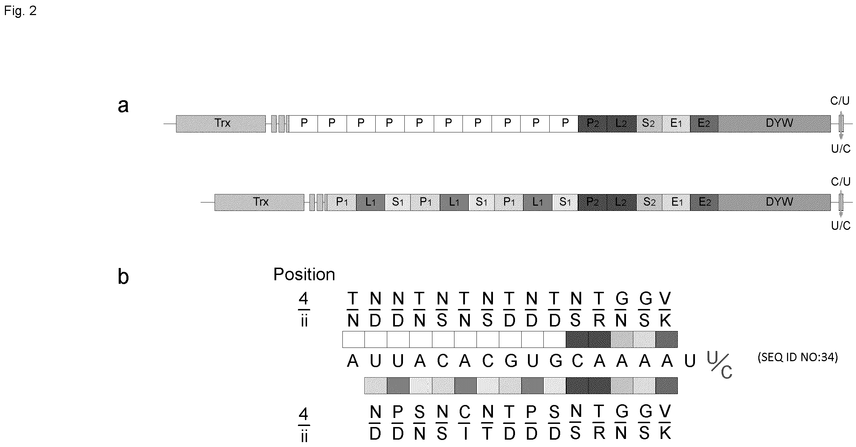

[Fig. 2] Constitution of the PPR protein designed in this study, (a) The PPR protein contains, from the N-terminal side, thioredoxin, His-tag and TEV site, followed by a PPR array consisting of P or PLS motifs, and finally, a DYW domain (DYW:PG, DYW:WW, or DYW:KP). The target sequence (including RNA editing site) of the PPR protein was inserted downstream of the termination codon. (b) The fourth and ii-th amino acids involved in RNA recognition within the P, P1, L1, S1 and P2 motifs. The fourth and ii-th amino acids in L2, S2, E1 and E2 are identical to those in CLB19 (Chateigner-Boutin, A.L., et al. (2008) Plant J., 56, 590-602).
[Fig. 3] RNA editing assay using *E. coli.* (a) Target base (C or T) on DNA of each PPR-DYW. (b) The C-to-U RNA editing activities of PPR-DYW:PG and WW, and (c) the U-to-C RNA editing activity of PPR-DYW:KP are shown. One of the results of three independent experiments is shown as an example. Average values of (d) C-to-U and (e) U-to-C RNA editing efficiencies measured three times (but twice for P-DYW:KP). The error bars indicate standard deviations.
[Fig. 4-1] Occurrence frequencies of amino acids in each selected DYW domain phylogenic group, visualized with sequence logos generated by using WebLogo. The same shall apply to Figs. 4-2 and 4-3. (a) DYW:PG.
[Fig. 4-2] (b) DYW:WW.
[Fig. 4-3] (c) DYW:KP.
[Fig. 5] RNA editing activities (C-to-U or U-to-C) in HEK293T cells. Sequencing results of target sites for PLS type fused with PG1 or WW1 domain (a), or KP1 domain (b). RNA editing activities obtained in three independent experiments (c). Bars indicate averages, and dots indicate RNA editing activities obtained in the experiments performed in triplicate. The left bar of each of the graphs indicates C-to-U editing activity, and the right bar of each of the graphs indicates U-to-C editing activity.
[Fig. 6] Improvement of KP domain performance by domain swapping. The RNA editing activity of a chimeric KP1a, where the center part containing the active site of KP1 (HxExₙCxxCH) was remained, and the domains in front of and behind the center part were swapped with the WW1 domain, was examined. A schematic diagram of the domain swapping (a), and actual RNA editing activity (b and c).
[Fig. 7] Measurement of RNA editing activities of KP domain mutants. RNA editing activities of KP1, KP2, KP3, and KP4 in *E. coli* (a), and HEK293T (b), and RNA editing activities of KP5 to KP23 in HEK293T (c).
[Fig. 8] Measurement of RNA editing activities of PG domain mutants. RNA editing activities of PG1 and PG2 in *E. coli* (a) and HEK293T (b), and RNA editing activities of PG3 to PG13 in HEK293T (c).
[Fig. 9] Measurement of RNA editing activities of WW domain mutants. RNA editing activities of WW1 and WW2 in *E. coli* (a) and HEK293T (b), and RNA editing activities of WW3 to WW14 in HEK293T (c).
[Fig. 10] Human mitochondria RNA editing. Target sequence information (a), and RNA editing activities (b and c).

### Modes for Carrying out the Invention

The present invention relates to a method for editing a target RNA, which comprises applying a DYW protein containing a DYW domain consisting of any one of the polypeptides a, b, c, and bc mentioned below to the target RNA:
a. a polypeptide having xₐ₁PGxₐ₂SWIExₐ₃-xₐ₁₆HP ... HxₐₐE ... Cxₐ₁₇Xa₁₈CH ... DYW, having a sequence identity of at least 40% to the sequence of SEQ ID NO: 1, and having a C-to-U/U-to-C editing activity,
b. a polypeptide having x_{b1}PGx_{b2}SWWTDx_{b3}-x_{b16}HP ... Hx_{bb}E ... Cx_{b17}x_{b18}CH ... DYW, having a sequence identity of at least 40% to the sequence of SEQ ID NO: 2, and having a C-to-U/U-to-C editing activity,
c. a polypeptide having KPAx_{c1}Ax_{c2}IEx_{c3} ... Hx_{cc}E ... Cx_{c4}x_{c5}CH ... x_{c6}x_{c7}x_{c8}, having a sequence identity of at least 40% to the sequence of SEQ ID NO: 3, and having a C-to-U/U-to-C editing activity, and
bc. a polypeptide having x_{b1}PGx_{b2}SWWTDx_{b3}-x_{b16}HP ... Hx_{cc}E ... Cx_{c4}x_{c5}CH ... Dx_{bc1}x_{bc2}, having a sequence identity of at least 40% to the sequence of SEQ ID NO: 90, and having a C-to-U/U-to-C editing activity.

In the present invention, C-to-U/U-to-C editing activity refers to an activity for converting an editing target C to U or an editing target U to C in a target RNA observed in an editing assay performed with a subject polypeptide bound to the C-terminal side of an RNA-binding domain that can sequence-specifically bind to the target RNA. The conversion is sufficient if at least about 3%, preferably about 5%, of the editing target bases are replaced with objective bases under appropriate conditions.

### [DYW domain]

All of xₐ₁PGxₐ₂SWIExₐ₃-xₐ₁₆HP ... HxₐₐE ... Cxₐ₁₇Xa₁₈CH ... DYW, x_{b1}PGx_{b2}SWWTDx_{b3}-x_{b16}HP ... H_{Xbb}E ... Cx_{b17}x_{b18}CH ... DYW, and KPAx_{c1}Ax_{c2}IEx_{c3} ... Hx_{cc}E ... Cx_{c4}x₅CH ... x_{c6}x_{c7}x_{c8} represent an amino acid sequence. In the sequences, x independently represents an arbitrary amino acid, and ... independently represents an arbitrary polypeptide fragment of an arbitrary length. In the present invention, the DYW domain can be represented by any one of these three amino acid sequences. In particular, a DYW domain consisting of xₐ₁PGxₐ₂SWIExₐ₃-xₐ₁₆HP ... HxₐₐE ... Cxₐ₁₇x₁₈CH ... DYW may be referred to as DYW:PG, a DYW domain consisting of x_{b1}PGx_{b2}SWWTDX_{b3}-x_{b16}HP ... Hx_{bb}E ... Cx_{b17}x_{b18}CH ... DYW may be referred to as DYW:WW, and a DYW domain consisting of KPAx_{c1}Ax_{c2}IEx_{c3} ... Hx_{cc}E ... Cx_{c4}x_{c5}CH ... x_{c6}x_{c7}x_{c8} may be referred to as DYW:KP.

The DYW domain has three regions, a region comprising a PG box consisting of about 15 amino acids at the N-terminus, a zinc-binding domain at the center (HxExₙCxxCH, where xₙ is a sequence of an arbitrary number n of arbitrary amino acids), and DYW at the C-terminus. The zinc-binding domain can be further divided into HxE and CxxCH regions. These regions of each DYW domain can be represented as in the table mentioned below.

**[Table 1]**

| | Region comprising PG box | HxE region | CxxCH region | DYW |
|---|---|---|---|---|
| DYW:PG | xₐ₁PGxₐ₂SWIExₐ₃-xₐ₁₆HP | HxₐₐE | Cxₐ₁₇xₐ₁₈CH | DYW |
| DYW:WW | x_{b1}PGx_{b2}SWWTDx_{b3}-x_{b16}HP | Hx_{bb}E | Cx_{b17}x_{b18}CH | DYW |
| DYW:KP | KPAx_{c1}Ax_{c2}IEx_{c3} | Hx_{cc}E | Cx_{c4}x_{c5}CH | x_{c6}x_{c7}x_{c8} |

### (DYW:PG)

DYW:PG is a polypeptide consisting of xₐ₁PGxₐ₂SWIExₐ₃-xₐ₁₆HP ... HxₐₐE ... Cxₐ₁₇xₐ₁₈CH ... DYW. It is preferably a polypeptide having xₐ₁PGxₐ₂SWIExₐ₃xₐ₁₆HP ... HxₐₐE ... Cxₐ₁₇Xa₁₈CH ... DYW, having a sequence identity (detailed in the section of [Terminology]) to the sequence of SEQ ID NO: 1, and having a C-to-U /U-to-C editing activity. DYW:PG has an activity to convert an editing target C to U (C-to-U editing activity). As SEQ ID NO: 1, a sequence of DYW:PG consisting of 136 amino acids in full length is shown, which was used in the experiments shown in the section of Examples of this description. This sequence is disclosed for the first time by this description, and is novel.

The full length of DYW:PG is not particularly limited so long as it can exhibit an C-to-U editing activity, but it is, for example, 110 to 160 amino acid length, preferably 124 to 148 amino acid length, more preferably 128 to 144 amino acid length, still more preferably 132 to 140 amino acid length.

In the region comprising the PG box of DYW:PG (xₐ1PGxₐ₂SWIExₐ₃-xₐ₁₆HP):
xₐ₁ is not particularly limited so long as DYW:PG can exhibit a C-to-U editing activity, but is preferably E (glutamic acid) or an amino acid similar thereto in nature, more preferably E,
xₐ₂ is not particularly limited so long as DYW:PG can exhibit a C-to-U editing activity, but is preferably C (cysteine) or an amino acid similar thereto in nature, more preferably C, and
the amino acids of xₐ₃ to xₐ₁₆ are not particularly limited so long as DYW:PG can exhibit a C-to-U editing activity, but are preferably the same as or similar in nature to the corresponding amino acids at positions 9 to 22 of the sequence of SEQ ID NO: 1, respectively, more preferably the same amino acids as the corresponding amino acids at positions 9 to 22 of the sequence of SEQ ID NO: 1, respectively.

In one of the preferred embodiments, the HxE region of DYW:PG is HSE, whatever the other regions may be.

In the CxxCH region of DYW:PG, i.e., Cxₐ₁₇xₐ₁₈CH:
xₐ₁₇ is not particularly limited so long as DYW:PG can exhibit a C-to-U editing activity, but is preferably G (glycine) or an amino acid similar thereto in nature, more preferably G, and
xₐ₁₈ is not particularly limited so long as DYW:PG can exhibit a C-to-U editing activity, but is preferably D (aspartic acid) or an amino acid similar thereto in nature, more preferably D.

In DYW:PG, the ... part binding the region containing the PG box and HxₐₐE, the ... part binding the HxₐₐE region and the CxxCH region, and the ... part linking the CxxCH region and DYW are referred to as first linkage part, second linkage part, and third linkage part, respectively (the same shall apply to the other DYW domains).

The full length of the first linkage part of DYW:PG is not particularly limited so long as DYW:PG can exhibit a C-to-U editing activity, but it is, for example, 39 to 47 amino acid length, preferably 40 to 46 amino acid length, more preferably 41 to 45 amino acid length, further preferably 42 to 44 amino acid length. The amino acid sequence of the first linkage part is not particularly limited so long as DYW:PG can exhibit a C-to-U editing activity, but is preferably the same as the part of positions 25 to 67 of the sequence of SEQ ID NO: 1, or is a sequence derived from that partial sequence by substitution, deletion, or addition of 1 to 22 amino acids, or a sequence having a sequence identity to the partial sequence, more preferably the same sequence as the partial sequence.

One of the preferred embodiments of the first linkage part of DYW:PG is a polypeptide having 43 amino acid length, and represented by the following formula, whatever the sequences of the other parts of the DYW domain may be:

Nₐ₂₅-Nₐ₂₆-Nₐ₂₇- ... -Nₐ₆₅-Nₐ₆₆-Nₐ₆₇

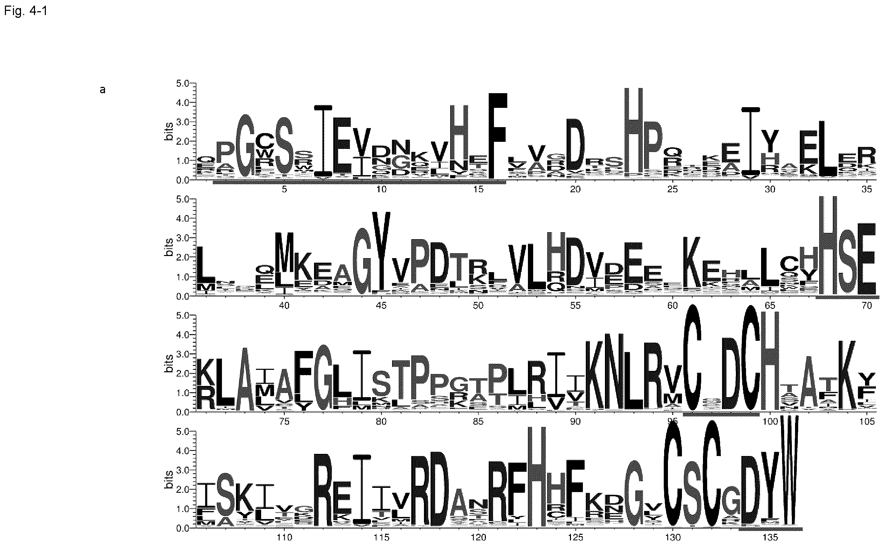

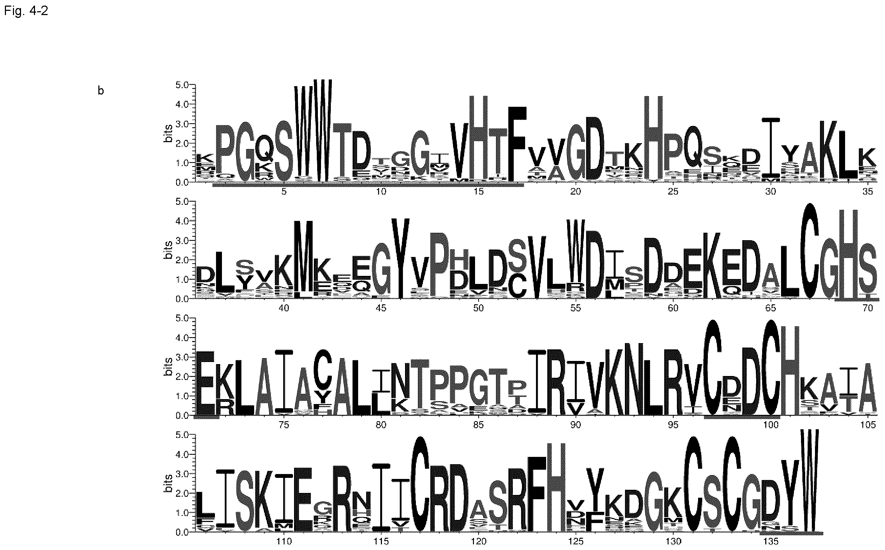

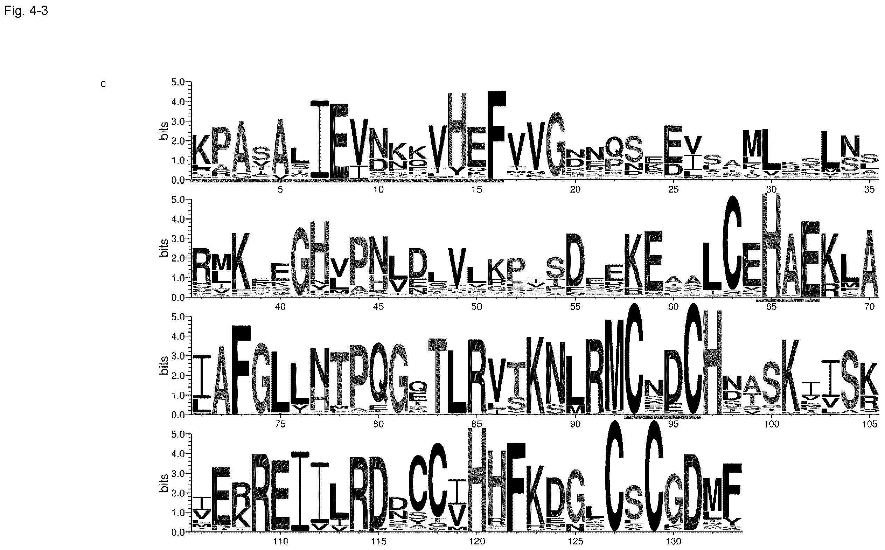

The above polypeptide is preferably the same as the part of positions 25 to 67 of the sequence of SEQ ID NO: 1, or a sequence that is derived from that partial sequence by substitution of a plurality of amino acids so that DYW:PG can exhibit a C-to-U editing activity. The amino acid substitution in this case is preferably designed so that the amino acids with large bits values at the corresponding positions in Fig. 4 (e.g., Nₐ₂₉, Nₐ₃₀, Nₐ₃₂, Nₐ₃₃, Nₐ₃₅, Nₐ₃₆, Nₐ₄₀, Nₐ₄₄, Nₐ₄₅, Nₐ₄₇, Nₐ₄₈, Nₐ₅₂, Nₐ₅₃, Nₐ₅₄, Nₐ₅₅, Nₐ₅₈, Nₐ₆i_{>} Nₐ₆₅, Nₐ₆₇) are the same as in Fig. 4, and the amino acids to be replaced are selected from the other amino acids.

The full length of the second linkage part of DYW:PG is not particularly limited so long as DYW:PG can exhibit a C-to-U editing activity, but it is, for example, 21 to 29 amino acid length, preferably 22 to 28 amino acid length, more preferably 23 to 27 amino acid length, further preferably 24 to 26 amino acid length. The amino acid sequence of the second linkage part is not particularly limited so long as DYW:PG can exhibit a C-to-U editing activity, but it is preferably the same as the part of positions 71 to 95 of the sequence of SEQ ID NO: 1, a sequence derived from that partial sequence by substitution, deletion, or addition of 1 to 13 amino acids, or a sequence having a sequence identity to the partial sequence, more preferably the same sequence as the partial sequence.

One of the preferred embodiments of the second linkage part of DYW:PG is a polypeptide of 25 amino acid length represented by the following formula, whatever the sequences of the other parts of the DYW domain may be.

Nₐ₇₁-Nₐ₇₂-Na₇₃- ... -Nₐ₉₃-Nₐ₉₄-Nₐ₉₅

The above polypeptide is preferably the same as the part of positions 71 to 95 of the sequence of SEQ ID NO: 1, or a sequence that is derived from the partial sequence by substitution of a plurality of amino acids so that DYW:PG can exhibit a C-to-U editing activity. The amino acid substitution in this case is preferably designed so that the amino acids with large bits values at the corresponding positions in Fig. 4 (e.g., Nₐ₇₁, Nₐ₇₂, Nₐ₇₃, Nₐ₇₆, Nₐ₇₇, Nₐ₇₈, Nₐ₇₉, Nₐ₈₁, Nₐ₈₂, Nₐ₈₆, Nₐ₈₈, Nₐ₈₉, Nₐ₉₁, Nₐ₉₂, Nₐ₉₃, Nₐ₉₄) are the same as in Fig. 4, and the amino acids to be replaced are selected from the other amino acids.

The full length of the third linkage part of DYW:PG is not particularly limited so long as DYW:PG can exhibit a C-to-U editing activity, but it is, for example, 29 to 37 amino acid length, preferably 30 to 36 amino acid length, more preferably 31 to 35 amino acid length, further preferably 32 to 34 amino acid length. The amino acid sequence of the third linkage part is not particularly limited so long as DYW:PG can exhibit a C-to-U editing activity, but it is preferably the same as the part of positions 101 to 133 of the sequence of SEQ ID NO: 1, a sequence derived from that partial sequence by substitution, deletion, or addition of 1 to 17 amino acids, or a sequence having a sequence identity to the partial sequence, more preferably the same sequence as the partial sequence.

One of the preferred embodiments of the third linkage part of DYW:PG is a polypeptide of 33 amino acid length represented by the following formula, whatever the sequences of the other parts of the DYW domain may be.

Nₐ₁₀₁-Nₐ₁₀₂-Nₐ₁₀₃- ... -Nₐ₁₃₁-Nₐ₁₃₂-Nₐ₁₃₃

The above polypeptide is preferably the same as the part of positions 101 to 133 of the sequence of SEQ ID NO: 1, or a sequence derived from that partial sequence by substitution of a plurality of amino acids so that DYW:PG can exhibit a C-to-U editing activity. The amino acid substitution in this case is preferably designed so that the amino acids with large bits values at the corresponding positions in Fig. 4 (e.g., Nₐ₁₀₂, Nₐ₁₀₄, Nₐ₁₀₇, Nₐ₁₁₂, Nₐ₁₁₄, Nₐ₁₁₇, Nₐ₁₁₈, Nₐ₁₂₁, Nₐ₁₂₂, Nₐ₁₂₃, Nₐ₁₂₄, Nₐ₁₂₅, Nₐ₁₂₈, Nₐ₁₃₀, Nₐ₁₃₁, Nₐ₁₃₂) are the same as in Fig. 4, and the amino acids to be replaced are selected from the other amino acids.

The RNA editing activity can be improved by introducing a mutation into the PG domain consisting of the sequence of SEQ ID NO: 1. A preferred example of such a mutated domain is PG11 (polypeptide consisting of the amino acid sequence of SEQ ID NO: 50) shown in the section of Examples of this description.

### (DYW:WW)

DYW:WW is a polypeptide consisting of x_{b1}PGx_{b2}SWWTDx_{b3}-x_{b16}HP ... Hx_{bb}E ... Cx_{b17}x_{b18}CH ... DYW. It is preferably a polypeptide having x_{b1}PGx_{b2}SWWTDx_{b3}-x_{b16}HP ... Hx_{bb}E ... Cx_{b17}x_{b18}CH ... DYW, having a sequence identity to the sequence of SEQ ID NO: 2, and having a C-to-U/U-to-C editing activity. DYW:WW has the activity to convert an editing target C to U (C-to-U editing activity). As SEQ ID NO: 2, the sequence of DYW:WW having a full length of 137 amino acids, which was used in the experiments shown in the section of Examples of this description, is shown. This sequence is disclosed for the first time by this description, and is novel.

The full length of DYW:WW is not particularly limited so long as it can exhibit a C-to-U editing activity, but it is, for example, 110 to 160 amino acid length, preferably 125 to 149 amino acid length, more preferably 129 to 145 amino acid length, further preferably 133 to 141 amino acid length.

In the region containing the PG box of DYW:WW, i.e., x_{b1}PGx_{b2}SWWTDx_{b3}-X_{b16}HP, the part consisting of WTD may be WSD.

In the region containing the PG box of DYW:WW:
x_{b1} is not particularly limited so long as DYW:WW can exhibit a C-to-U editing activity, but is preferably K (lysine) or an amino acid similar thereto in nature, more preferably K,
x_{b2} is not particularly limited so long as DYW:WW can exhibit a C-to-U editing activity, but is preferably Q (glutamine) or an amino acid similar thereto in nature, more preferably Q, and
the amino acids of x_{b3} to x_{b16} are not particularly limited so long as DYW:WW can exhibit a C-to-U editing activity, but are preferably the same as or similar in nature to the corresponding amino acids at positions 10 to 23 of the sequence in SEQ ID NO: 2, respectively, more preferably the same amino acids as the corresponding amino acids at positions 10 to 23 of the sequence of SEQ ID NO: 2, respectively.

In one of the preferred embodiments, the HxE region of DYW:WW is HSE, whatever the sequences of the other parts may be.

In the CxxCH region of DYW:WW, i.e., Cx_{b17}x_{b18}CH:
x_{b17} is not particularly limited so long as DYW:WW can exhibit a C-to-U editing activity, but is preferably D (aspartic acid) or an amino acid similar thereto in nature, more preferably D, and
x_{b18} is not particularly limited so long as DYW:WW can exhibit a C-to-U editing activity, but is preferably D or an amino acid similar thereto in nature, more preferably D.

The full length of the first linkage part of DYW:WW is not particularly limited so long as DYW:WW can exhibit a C-to-U editing activity, but it is, for example, 39 to 47 amino acid length, preferably 40 to 46 amino acid length, more preferably 41 to 45 amino acid length, further preferably 42 to 44 amino acid length. The amino acid sequence of the first linkage part is not particularly limited so long as DYW:WW can exhibit a C-to-U editing activity, but it is preferably the same as the part of positions 25 to 67 of the sequence of SEQ ID NO: 2, a sequence derived from that partial sequence by substitution, deletion, or addition of 1 to 22 amino acids, or a sequence having a sequence identity to the partial sequence, more preferably the same sequence as the partial sequence.

One of the preferred embodiments of the first linkage part of DYW:WW is a polypeptide of 43 amino acid length represented by the following formula, whatever the sequences of the other parts of the DYW domain may be.

N_{b26}-N_{b27}-N_{b28}- ... -N_{b66}-N_{b67}-N_{b68}

The above polypeptide is preferably the same as the part of positions 26 to 68 of the sequence of SEQ ID NO: 2, or a sequence that is derived from that partial sequence by substitution of a plurality of amino acids so that DYW:WW can exhibit a C-to-U editing activity. The amino acid substitution in this case is preferably designed so that the amino acids with large bits values at the corresponding positions in Fig. 4 (e.g., N_{b26}, N_{b30}, N_{b33}, N_{b34}, N_{b37}, N_{b4}), N_{b45}, N_{b46}, N_{b48}, N_{b49}, N_{b51}, N_{b52}, N_{b53}, N_{b55}, N_{b56}, N_{b57}, N_{b59}, N_{b61}, N_{b62}, N_{b63}, N_{b64}, N_{b66}, N_{b67}, N_{b68}) are the same as in Fig. 4, and the amino acids to be replaced are selected from the other amino acids.

The full length of the second linkage part of DYW:WW is not particularly limited so long as DYW:WW can exhibit a C-to-U editing activity, but it is, for example, 21 to 29 amino acid length, preferably 22 to 28 amino acid length, more preferably 23 to 27 amino acid length, further preferably 24 to 26 amino acid length. The amino acid sequence of the second linkage part is not particularly limited so long as DYW:WW can exhibit a C-to-U editing activity, but it is preferably the same as the part of positions 71 to 95 of the sequence of SEQ ID NO: 2, a sequence derived from that partial sequence by substitution, deletion, or addition of 1 to 13 amino acids, or a sequence having a sequence identity to the partial sequence, more preferably the same sequence as the partial sequence.

One of the preferred embodiments of the second linkage part of DYW:WW is a polypeptide of 25 amino acid length represented by the following formula, whatever the sequences of the other parts of the DYW domain may be.

N_{b72}-N_{b73}-N_{b74}- ... -N_{b94}-N_{b95}-N_{b96}

The above polypeptide is preferably the same as the part of positions 72 to 96 of the sequence of SEQ ID NO: 2, or a sequence derived from that partial sequence by substitution of a plurality of amino acids so that DYW:WW can exhibit a C-to-U editing activity. The amino acid substitution in this case is preferably designed so that the amino acids with large bits values at the corresponding positions in Fig. 4 (e.g., N_{b72}, N_{b73}, N_{b74}, N_{b75}, N_{b77}, N_{b78}, N_{b79}, N_{b81}, N_{b82}, N_{b84}, N_{b88}, N_{b89}, N_{b90}, N_{b91}, N_{b92}, N_{b93}, N_{b94}, N_{b95}, N_{b96}) are the same as in Fig. 4, and the amino acids to be replaced are selected from the other amino acids.

The full length of the third linkage part of DYW:WW is not particularly limited so long as DYW:WW can exhibit a C-to-U editing activity, but it is, for example, 29 to 37 amino acid length, preferably 30 to 36 amino acid length, more preferably 31 to 35 amino acid length, further preferably 32 to 34 amino acid length. The amino acid sequence of the third linkage part is not particularly limited so long as DYW:WW can exhibit a C-to-U editing activity, but it is preferably the same as the part of positions 101 to 133 of the sequence of SEQ ID NO: 2, a sequence derived from that partial sequence by substitution, deletion, or addition of 1 to 17 amino acids, or a sequence having a sequence identity to the partial sequence, more preferably the same sequence as the partial sequence.

One of the preferred embodiments of the third linkage part of DYW:WW is a polypeptide of 33 amino acid length represented by the following formula, whatever the sequences of the other parts of the DYW domain may be.

N_{b102}-N_{b103}-N_{b104}- ... -N_{b132}-N_{b133}-N_{b134}

The above polypeptide is preferably the same as the part of positions 102 to 134 of the sequence of SEQ ID NO: 2, or a sequence derived from that partial sequence by substitution of a plurality of amino acids so that DYW:WW can exhibit a C-to-U editing activity. The amino acid substitution in this case is preferably designed so that the amino acids with large bits values at the corresponding positions in Fig. 4 (e.g., N_{b104}, N_{b105}, N_{b107}, N_{b108}, N_{b109}, N_{b110}, N_{b111}, N_{b113}, N_{b115}, N_{b116}, N_{b117}, N_{b118}, N_{b119}, N_{b121}, N_{b122}, N_{b123}, N_{b124}, N_{b126}, N_{b129}, N_{b131}, N_{b132}, N_{b133}, N_{b134}) are the same as in Fig. 4, and the amino acids to be replaced are selected from the other amino acids.

The RNA editing activity can be improved by introducing a mutation into the WW domain consisting of the sequence of SEQ ID NO: 2. Preferred examples of the domain to which such a mutation has been introduced are WW2 to WW11 and WW13 shown in the section of Examples of this description, and WW11 (polypeptide consisting of the amino acid sequence of SEQ ID NO: 63) shows particularly high editing activity.

### (DYW:KP)

DYW:KP is a polypeptide consisting of KPAx_{c1}Ax_{c2}IEx_{c3} ... Hx_{cc}E ... Cx_{c4}x_{c5}CH ... x_{c6}x_{c7}x_{c8}. It is preferably a polypeptide having KPAx_{c1}Ax_{c2}IEx_{c3} ... Hx_{cc}E ... Cx_{c4}x_{c5}CH ... x_{c6}x_{c7}x_{c8}, having a sequence identity to the sequence of SEQ ID NO: 3, and having a C-to-U/U-to-C editing activity. DYW:KP has an activity to convert an editing target U to C (U-to-C editing activity). As SEQ ID NO: 3, the sequence of DYW:KP having a full length of 133 amino acids is shown, which was used in the experiments described in the section of Examples of this description. This sequence is disclosed for the first time by this description, and is novel.

The full length of DYW:KP is not particularly limited so long as it can exhibit a U-to-C editing activity, but it is, for example, 110 to 160 amino acid length, preferably 121 to 145 amino acid length, more preferably 125 to 141 amino acid length, further preferably 129 to 137 amino acid length.

In the region containing the PG box of DYW:KP, namely, KPAx_{c1}Ax_{c2}IEx_{c3}:
x_{c1} is not particularly limited so long as DYW:KP can exhibit a U-to-C editing activity, but is preferably S (serine) or an amino acid similar thereto in nature, more preferably S,
x_{c2} is not particularly limited so long as DYW:KP can exhibit a U-to-C editing activity, but is preferably L (leucine) or an amino acid similar thereto in nature, more preferably L, and
x_{c3} is not particularly limited so long as DYW:KP can exhibit a U-to-C editing activity, but is preferably V (valine) or an amino acid similar thereto in nature, more preferably V.

In one of the preferred embodiments, the HxE region of DYW:KP is HAE, whatever the sequences of the other parts may be.

In the CxxCH region of DYW:KP, i.e., Cx_{c4}x_{c5}CH:
x_{c4} is not particularly limited so long as DYW:KP can exhibit a U-to-C editing activity, but is preferably N (asparagine) or an amino acid similar thereto in nature, more preferably N, and
x_{b5} is not particularly limited so long as DYW:KP can exhibit a U-to-C editing activity, but is preferably D (aspartic acid) or an amino acid similar thereto in nature, more preferably D.

In the part of DYW:KP corresponding to DYW, i.e., x_{c6}x_{c7}x_{c8}:
x_{c6} is not particularly limited so long as DYW:KP can exhibit a U-to-C editing activity, but is preferably D (aspartic acid) or an amino acid similar thereto in nature, more preferably D,
x_{c7} is not particularly limited so long as DYW:KP can exhibit a U-to-C editing activity, but is preferably M (methionine) or an amino acid similar thereto in nature, more preferably M, and
x_{b8} is not particularly limited so long as DYW:KP can exhibit a U-to-C editing activity, but is preferably F (phenylalanine) or an amino acid similar thereto in nature, more preferably F.

In one of the preferred embodiments, x_{c6}x_{c7}x_{c8} is Dx_{c7}x_{c8}, whatever the sequences of the other parts may be.

In another one of the preferred embodiments, x_{c6}x_{c7}x_{c8} is GRP, whatever the sequences of the other part may be.

The full length of the first linkage part of DYW:KP is not particularly limited so long as DYW:KP can exhibit a U-to-C editing activity, but it is, for example, 51 to 59 amino acid length, preferably 52 to 58 amino acid length, more preferably 53 to 57 amino acid length, further preferably 54 to 56 amino acid length. The amino acid sequence of the first linkage part is not particularly limited so long as DYW:KP can exhibit a U-to-C editing activity, but is preferably the same as the part of positions 10 to 64 of the sequence of SEQ ID NO: 3, a sequence derived from that partial sequence by substitution, deletion, or addition of 1 to 28 amino acids, or a sequence having a sequence identity to that partial sequence, more preferably the same sequence as the partial sequence.

One of the preferred embodiments of the first linkage part of DYW:KP is a polypeptide of 55 amino acid length represented by the following formula, whatever the sequences of the other parts of the DYW domain may be.

N_{c10}-N_{c11}-N_{c12}- ... -N_{c62}-N_{c63}-N_{c64}

The above polypeptide is preferably the same as the part of positions 10 to 64 of the sequence of SEQ ID NO: 3, or a sequence derived from that partial sequence by substitution of a plurality of amino acids so that DYW:KP can exhibit a U-to-C editing activity. The amino acid substitution in this case is preferably designed so that the amino acids with large bits values at the corresponding positions in Fig. 4 (e.g., N_{c10}, N_{c13}, N_{c14}, N_{c15}, N_{c16}, N_{c17}, N_{c18}, N_{c19}, N_{c25}, N_{c26}, N_{c29}, N_{c30}, N_{c33}, N_{c34}, N_{c36}, N_{c38}, N_{c41}, N_{c42}, N_{c44}, N_{c45}, N_{c47}, N₄₉, N_{c55}, N_{c58}, N_{c59}, N_{c62}, N_{c63}, N_{c64}) are the same as in Fig. 4, and the amino acids to be replaced are selected from the other amino acids.

The full length of the second linkage part of DYW:KP is not particularly limited so long as DYW:KP can exhibit a U-to-C editing activity, but it is, for example, 21 to 29 amino acid length, preferably 22 to 28 amino acid length, more preferably 23 to 27 amino acid length, further preferably 24 to 26 amino acid length. The amino acid sequence of the second linkage part is not particularly limited so long as DYW:KP can exhibit a U-to-C editing activity, but it is preferably the same as the part of positions 68 to 92 of the sequence of SEQ ID NO: 3, a sequence derived from that partial sequence by substitution, deletion, or addition of 1 to 13 amino acids, or a sequence having a sequence identity to that partial sequence, more preferably the same sequence as the partial sequence.

One of the preferred embodiments of the second linkage part of DYW:KP is a polypeptide of 25 amino acid length represented by the following formula, whatever the sequences of the other parts of the DYW domain may be.

N_{c68}-N_{c69}-N_{c70}- ... -N_{c90}-N_{c91}-N_{c92}

The above polypeptide is preferably the same as the part of positions 68 to 92 of the sequence of SEQ ID NO: 3, or a sequence derived from that partial sequence by substitution of a plurality of amino acids so that DYW:KP can exhibit a U-to-C editing activity. The amino acid substitution in this case is preferably designed so that the amino acids with large bits values at the corresponding positions in Fig. 4 (e.g., N_{c68}, N_{c70}, N_{c71}, N_{c72}, N_{c73}, N_{c74}, N_{c75}, N_{c76}, N_{c77}, N_{c78}, N_{c79}, N_{c80}, N_{c81}, N_{c83}, N_{c84}, N_{c85}, N_{c86}, N_{c87}, N_{c88}, N_{c89}, N_{c90}, N_{c91}, N_{c92}) are the same as in Fig. 4, and the amino acids to be replaced are selected from the other amino acids.

The full length of the third linkage part of DYW:KP is not particularly limited so long as DYW:KP can exhibit a U-to-C editing activity, but it is, for example, 29 to 37 amino acid length, preferably 30 to 36 amino acid length, more preferably 31 to 35 amino acid length, further preferably 32 to 34 amino acid length. The amino acid sequence of the third linkage is not particularly limited so long as DYW:KP can exhibit a U-to-C editing activity, but it is preferably the same as the part of positions 98 to 130 of the sequence of SEQ ID NO: 3, a sequence derived from that partial sequence by substitution, deletion, or addition of 1 to 17 amino acids, or a sequence having a sequence identity to the partial sequence, more preferably the same sequence as the partial sequence.

One of the preferred embodiments of the third linkage part of DYW:KP is a polypeptide of 33 amino acid length represented by the following formula, whatever the sequences of the other parts of the DYW domain may be.

N_{c98}-N_{c99}-N_{c100}- ... -N_{c128}-N_{c129}-N_{c130}

The above polypeptide is preferably the same as the part of positions 98 to 130 of the sequence of SEQ ID NO: 3, or a sequence derived from that partial sequence by substitution of a plurality of amino acids so that DYW:KP can exhibit a U-to-C editing activity. The amino acid substitution in this case is preferably designed so that the amino acids with large bits values at the corresponding positions in Fig. 4 (e.g., N_{c98}, N_{c100}, N_{c101}, N_{c103}, N_{c104}, N_{c105}, N_{c107}, N_{c109}, N_{c110}, N_{c111}, N_{c112}, N_{c114}, N_{c115}, N_{c117}, N_{c118}, N_{c119}, N_{c120}, N_{c121}, N_{c122}, N_{c123}, N_{c124}, N_{c125}, N_{c127}, N_{c129}, N_{c130}) are the same as in Fig. 4, and the amino acids to be replaced are selected from the other amino acids.

The editing activity can be improved by introducing a mutation into the KP domain consisting of the sequence of SEQ ID NO: 3. Preferred examples of the domain to which such a mutation has been introduced are KP2 to KP23 (SEQ ID NOS: 68 to 89) shown in the section of Examples of this description. KP22 (SEQ ID NO: 88) has the highest U-to-C editing activity, and low C-to-U editing activity, and thus the RNA editing activity thereof is improved compared with the KP domain consisting of the sequence of SEQ ID NO: 3.

### (Chimeric DYW)

The DYW domain can be divided into several regions on the basis of the conservation of amino acid sequence. A chimeric DYW in which these regions are exchanged may have an improved C-to-U or U-to-C editing activity. One preferred embodiment is one consisting of the PG box-containing region of DYW:WW, i.e., x_{b1}PGx_{b2}SWWTDx_{b3}-x_{b16}HP and DYW fused with the other regions of DYW:KP (... Hx_{cc}E ... Cx_{c4}x_{c5}CH ...). In the formulas, x_{b1}, x_{b2}, and x_{b3} to x_{b16} are as described above for DYW:WW. The first, second, and third linkage parts are as described above for DYW:KP. The DYW part may be Dx_{bc1}x_{bc2}. The full length is not particularly limited so long as it can exhibit a U-to-C editing activity, but it is, for example, 110 to 160 amino acid length, preferably 125 to 149 amino acid length, more preferably 129 to 145 amino acid length, further preferably 133 to 141 amino acid length.

One of preferred chimeric domains consists of a polypeptide having x_{b1}PGx_{b2}SWWTDx_{b3}-x_{b16}HP ... Hx_{cc}E ... Cx_{c4}x_{c5}CH ... Dx_{bc1}x_{bc2}, having a sequence identity of at least 40% to the sequence of SEQ ID NO: 90, and having a C-to-U/U-to-C editing activity.

One of particularly preferred chimeric domains is a polypeptide having a sequence identity to the sequence of SEQ ID NO: 90, and having a U-to-C editing activity. As SEQ ID NO: 90, the sequence of the chimeric domain used in the experiments described in the section of Examples of this description is shown. This domain shows higher U-to-C editing activity compared with DYW:KP consisting of the sequence of SEQ ID NO: 3, and shows almost no C-to-U editing activity.

### (Comparison with known sequences)

As described above, the DYW domains consisting of the sequences of SEQ ID NOS: 1, 2, and 3 are novel. The results of research performed by using a PPR database (https://ppr.plantenergy.uwa.edu.au/onekp/, Non-patent document 11 mentioned above) are shown below.

**[Table 2-1]**

| DYW:PG (SEQ ID NO: 1) | | |
|---|---|---|
| Sequence Code | Origin | Identity |
| - PYHZ-2068577-R | Isoetes, lycophyte | 78% |
| - ENQF-2003735-R | Lycopodium annotinum, lycophyte | |

| DYW:WW (SEQ ID NO: 2) | | |
|---|---|---|
| - ANON-2000164-R | Leiosporoceros dussii, hornwort | 84% |
| - ANON-2000163-R | | |
| - ANON-2004532-F | | |
| - ANON-2001227-F | | |

| DYW:KP (SEQ ID NO: 3) | | |
|---|---|---|
| - UWOD-2140463-R | Plagiogyria japonica, fern | 86% |

Therefore, the present invention also provides a polypeptide, which is any one of the following polypeptides d to i:
d. a polypeptide having a sequence identity higher than 78%, preferably that of 80% or higher, more preferably that of 85% or higher, further preferably that of 90%, further preferably that of 95%, further preferably that of 97%, to the sequence of SEQ ID NO: 1, and having a C-to-U editing activity;
e. a polypeptides having a sequence identity higher than 84%, preferably that of 85% or higher, more preferably that of 90% or higher, further preferably that of 95%, further preferably that of 97%, to the sequence of SEQ ID NO: 2, and having a C-to-U editing activity;
f. a polypeptides having a sequence identity higher than 86%, preferably that of 87% or higher, more preferably that of 90% or higher, further preferably that of 95%, further preferably that of 97%, to the sequence of SEQ ID NO: 3, and having a U-to-C editing activity;
g. a polypeptides having a sequence derived from the sequence of SEQ ID NO: 1 by substitution, deletion, or addition of 1 to 29, preferably 1 to 25, more preferably 1 to 21, further preferably 1 to 17, further preferably 1 to 13, further preferably 1 to 9, further preferably 1 to 5, of amino acids, and having a C-to-U editing activity;
h. a polypeptide having a sequence derived from the sequence of SEQ ID NO: 2 by substitution, deletion, or addition of 1 to 21, preferably 1 to 18, more preferably 1 to 15, further preferably 1 to 12, further preferably 1 to 9, further preferably 1 to 6, of amino acids, and having a C-to-U editing activity; and
i. a polypeptide having a sequence derived from the sequence of SEQ ID NO: 3 by substitution, deletion, or addition of 1 to 18, preferably 1 to 16, more preferably 1 to 14, further preferably 1 to 12, further preferably 1 to 10, further preferably 1 to 8, further preferably 1 to 6, of amino acids, and having a U-to-C editing activity.

The alignment of these sequences is shown below. The alignment was prepared by using AliView (Larsson, A. (2014) AliView: a fast and lightweight alignment viewer and editor for large data sets, Bioinformatics, 30(22):3276-3278, http://dx.doi.org/10.1093/bioinformatics/btu531). Identical amino acids are indicated with dots.

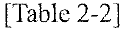

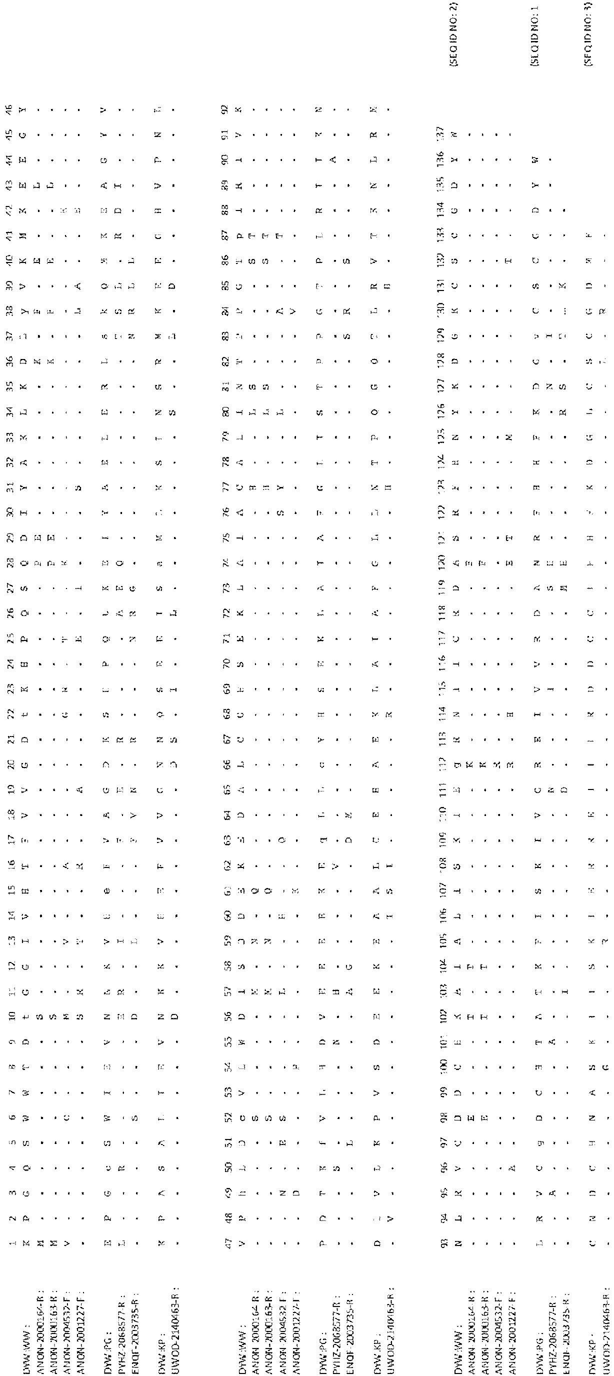

### [RNA-binding domain]

In the present invention, an RNA binding protein is used as an RNA-binding domain in order to target RNA containing an editing target when the editing target is converted by using the DYW domain.

A preferred example of such an RNA binding protein used as an RNA-binding domain is a PPR protein constituted by a PPR motif.

### (PPR motif)

The "PPR motif' means a polypeptide constituted with 30 to 38 amino acids and having an amino acid sequence that has, when the amino acid sequence is analyzed with a protein domain search program on the web, an E value not larger than a predetermined value (desirably E-03) obtained as PF01535 in the case of Pfam, or PS51375 in the case of Prosite PPR. The position numbers of the amino acids constituting the PPR motif defined in the present invention are substantially synonymous with those of PF01535, while they correspond to numbers obtained by subtracting 2 from the position numbers of the amino acids in PS51375 (e.g., No. 1 defined in the present invention is No. 3 in PS51375). However, the "ii"(-2) amino acid means, among the amino acids constituting the PPR motif, the second amino acid counted from the last amino acid (C-terminus side), or the second amino acid backed from the first amino acid in the next PPR motif towards the N-terminus side, i.e., -2nd amino acid. When the next PPR motif is not clearly identified, the second amino acid backed from the first amino acid of the next helix structure is represented as "ii". For Pfam, http://pfam.sanger.ac.uk/ can be referred to, and for Prosite, http://www.expasy.org/prosite/ can be referred to.

The conserved amino acid sequences of the PPR motifs are poorly conserved at the amino acid level, but the two α-helices are well conserved in the secondary structure. Typical PPR motifs consist of 35 amino acids, but lengths thereof are variable, ranging from 30 to 38 amino acids.

More specifically, the PPR motifs consist of a polypeptide of 30 to 38 amino acid length represented by the formula 1.
[Formula 1]

**(Helix A)-X-(Helix** B)-L (Formula 1)

In the formula:
Helix A is a part of 12 amino acid length capable of forming an α-helix structure, and is represented by the formula 2;
[Formula 2]

**A₁-A₂-A₃-A₄-A₅-A₆-A₇-A₈-A₉-A₁₀-A₁₁-A₁₂** (Formula 2)

wherein, in the formula 2, A₁ to A₁₂ independently represent an amino acid;
X does not exist, or is a part of 1 to 9 amino acid length;
Helix B is a part of 11 to 13 amino acid length capable of forming an α-helix structure; and
L is a part of 2 to 7 amino acid length represented by the formula 3;
[Formula 3]

**Lᵥᵢᵢ-Lᵥᵢ-Lᵥ-Lᵢᵥ-Lᵢᵢᵢ-Lᵢᵢ-Lᵢ** (Formula 3)

wherein, in the formula 3, the amino acids are numbered "i" (-1), "ii" (-2), and so on from the C-terminus side,
provided that Lᵢᵢᵢ to Lᵥᵢᵢ may not exist.

### (PPR code)

The combinations of three amino acids at positions 1, 4, and ii of PPR motifs are important for specific binding thereof to a base, and the type of base to which PPR motifs bind can be determined by the combinations. The relationship between the combinations of the amino acids of position 1, 4, and ii and bases to which they can bind is known as the PPR codes (Patent document 2 mentioned above), and is as follows.
(1) When the combination of the three amino acids of A₁, A₄, and Lᵢᵢ consists of valine, asparagine, and aspartic acid in this order, the PPR motif has such a selective RNA base-binding ability that the motif strongly binds to U, less strongly to C, and still less strongly to A or G.
(2) When the combination of the three amino acids of A₁, A₄, and Lᵢᵢ consists of valine, threonine, and asparagine in this order, the PPR motif has such a selective RNA base-binding ability that the motif strongly binds to A, less strongly to G, and still less strongly to C, but dose not bind to U.
(3) When the combination of the three amino acids of A₁, A₄, and Lᵢᵢ consists of valine, asparagine, and asparagine in this order, the PPR motif has such a selective RNA base-binding ability that the motif strongly binds to C, and less strongly to A or U, but does not bind to G.
(4) When the combination of the three amino acids of A₁, A₄, and Lᵢᵢ consists of glutamic acid, glycine, and aspartic acid in this order, the PPR motif has such a selective RNA base-binding ability that the motif strongly binds to G, but does not bind to A, U, and C.
(5) When the combination of the three amino acids of A₁, A₄, and Lᵢᵢ consists of isoleucine, asparagine, and asparagine in this order, the PPR motif has such a selective RNA base-binding ability that the motif strongly binds to C, less strongly to U, and still less strongly to A, but does not bind to G.
(6) When the combination of the three amino acids of A₁, A₄, and Lᵢᵢ consists of valine, threonine, and aspartic acid in this order, the PPR motif has such a selective RNA base-binding ability that the motif strongly binds to G, and less strongly to U, but does not bind to A and C.
(7) When the combination of the three amino acids of A₁, A₄, and Lᵢᵢ consists of lysine, threonine, and aspartic acid in this order, the PPR motif has such a selective RNA base-binding ability that the motif strongly binds to G, and less strongly to A, but does not bind to U and C.
(8) When the combination of the three amino acids of A₁, A₄, and Lᵢᵢ consists of phenylalanine, serine, and asparagine in this order, the PPR motif has such a selective RNA base-binding ability that the motif strongly binds to A, less strongly to C, and still less strongly to G and U.
(9) When the combination of the three amino acids of A₁, A₄, and Lᵢᵢ consists of valine, asparagine, and serine in this order, the PPR motif has such a selective RNA base-binding ability that the motif strongly binds to C, and less strongly to U, but does not bind to A and G.
(10) When the combination of the three amino acids of A₁, A₄, and Lᵢᵢ consists of phenylalanine, threonine, and asparagine in this order, the PPR motif has such a selective RNA base-binding ability that the motif strongly binds to A, but does not bind to G, U, and C.
(11) When the combination of the three amino acids of A₁, A₄, and Lᵢᵢ consists of isoleucine, asparagine, and aspartic acid in this order, the PPR motif has such a selective RNA base-binding ability that the motif strongly binds to U, and less strongly to A, but does not bind to G and C.
(12) When the combination of the three amino acids of A₁, A₄, and Lᵢᵢ consists of threonine, threonine, and asparagine in this order, the PPR motif has such a selective RNA base-binding ability that the motif strongly binds to A, but does not bind to G, U, and C.
(13) When the combination of the three amino acids of A₁, A₄, and Lᵢᵢ consists of isoleucine, methionine, and aspartic acid in this order, the PPR motif has such a selective RNA base-binding ability that the motif strongly binds to U, and less strongly to C, but does not bind to A and G.
(14) When the combination of the three amino acids of A₁, A₄, and Lᵢᵢ consists of phenylalanine, proline, and aspartic acid in this order, the PPR motif has such a selective RNA base-binding ability that the motif strongly binds to U, and less strongly to C, but does not bind to A and G.
(15) When the combination of the three amino acids of A₁, A₄, and Lᵢᵢ consists of tyrosine, proline, and aspartic acid in this order, the PPR motif has such a selective RNA base-binding ability that the motif strongly binds to U, but does not bind to A, G, and C.
(16) When the combination of the three amino acids of A₁, A₄, and Lᵢᵢ consists of leucine, threonine, and aspartic acid in this order, the PPR motif has such a selective RNA base-binding ability that the motif strongly binds to G, but does not bind to A, U, and C.

### (P Array)

The PPR proteins are classified into two families, P and PLS, according to the structure of the constituent PPR motifs.

P-type PPR proteins consist of simple repeat (P array) of the standard 35 amino acids PPR motif (P). The DYW domain of the present invention can be used in a state that it is linked to a P-type PPR protein.

### (PLS array)

The sequence of PPR motifs in PLS-type PPR proteins is composed of repeating units of three PPR motifs, P1, L1, and S1, followed by P2, L2, and S2 motifs on the C-terminal side of the repeat. In addition, two PPR-like motifs called E1 and E2, and the DYW domain may follow the C-terminal side of the last P2L2S2 motif.

The full length of P1 is not particularly limited so long as the protein can bind to a target base, but it is, for example, 33 to 37 amino acid length, preferably 34 to 36 amino acid length, more preferably 35 amino acid length. The full length of L1 is not particularly limited so long as the protein can bind to a target base, but it is, for example, 33 to 37 amino acid length, preferably 34 to 36 amino acid length, more preferably 35 amino acid length. The full length of S1 is not particularly limited so long as the protein can bind to a target base, but it is, for example, 30 to 33 amino acid length, preferably 30 to 32 amino acid length, more preferably 31 amino acid length.

The full length of P2 is not particularly limited so long as the protein can bind to the target base, but it is, for example, 33 to 37 amino acid length, preferably 34 to 36 amino acid length, more preferably 35 amino acid length. The full length of L2 is not particularly limited as long as the protein can bind to the target base, but it is, for example, 34 to 38 amino acid length, preferably 35 to 37 amino acid length, more preferably 36 amino acid length. The full length of S2 is not particularly limited so long as the protein can bind to the target base, but it is, for example, 30 to 34 amino acid length, preferably 31 to 33 amino acid length, more preferably 32 amino acid length. As SEQ ID NOS: 17, 22, and 27, the sequences of P2 used in the section of Examples of this description are shown. As SEQ ID NOS: 18, 23, and 28, the sequences of L2 used in the section of Examples of this description are shown. As SEQ ID NOS: 19, 24, and 29, the sequences of S2 used in the section of Examples of this description are shown.

The full length of E1 is not particularly limited so long as the protein can bind to the target base, but it is, for example, 32 to 36 amino acid length, preferably 33 to 35 amino acid length, more preferably 34 amino acid length. As SEQ ID NOS: 20, 25 and 30, the sequences of E1 used in the section of Examples of this description are shown.

The full length of E2 is not particularly limited so long as the protein can bind to the target base, but it is, for example, 30 to 34 amino acid length, preferably 31 to 33 amino acid length, more preferably 33 amino acid length. As SEQ ID NOS: 21, 26 and 31, the sequences of E2 used in the section of Examples of this description are shown.

In PLS-type PPR proteins, the repeat of P1L1S1 and the part up to P2 can be designed according to the sequence of the target RNA, following the rules of the PPR codes described above.

The S2 motif correlates with the nucleotides to which the amino acid at position ii (the 31st N in SEQ ID NO: 19) corresponds (Non-patent document 8 mentioned above). The S2 motif can be incorporated into PLS-type PPR proteins, noting that the fourth C or U, counted from the target base of the S2 motif towards the right side, is the target base for editing by the DYW domain.

In the E1 motif, correlation with nucleotides is found only for the fourth amino acid (fourth G in SEQ ID NO: 20) (Ruwe et al. (2019) New Phytol., 222 218-229).

The fourth and last amino acids in the E2 motif (fourth V and 33rd K in SEQ ID NO: 21, respectively) are highly conserved, and are not involved in specific PPR-RNA recognition (Non-patent document 2 mentioned above).

The number of repeats of P1L1S1 is not particularly limited so long as the protein can bind to the target sequence, but it is, for example, 1 to 5, preferably 2 to 4, more preferably 3. In principle, even one repeat unit (3 motifs) can be used. MEF8 consisting of 5 PPR motifs (L1-S1-P2-L2-S2-E-DYW) is known to be involved in editing of about 60 sites.

In natural PPR proteins, P1L1S1 locating at the beginning and end parts are different from the internal P1L1S1 in that they have distinct differences in amino acid residues at specific positions. From the viewpoint of designing an artificial PLS array as close as possible to a naturally occurring one, it is preferred that three types of P1L1S1 are designed according to the position in the PPR motif, i.e., as the first (N-terminal side) P1L1S1, internal P1L1S1, and the last (C-terminal side) P1L1S1 locating just before P2L2S2. In addition to those consisting of P-L-S repeating units, natural PPR proteins may contain repeat of SS (31 amino acids), and such PPR proteins can also be used in the present invention.

### [DYW protein]

The present invention provides a DYW protein for editing a target RNA, which comprises an RNA-binding domain that contains at least one PPR motif and can sequence-specifically bind to a target RNA according to the PPR code rules, and a DYW domain, which is one of the aforementioned DYW:PG, DYW:WW, and DYW:KP.

Such a DYW protein can be an artificial protein. Artificial means that the protein is not a natural product, but it is artificially synthesized. Being artificial means that, for example, the protein has a DYW domain with a sequence not found in nature, the protein has a PPR binding domain with a sequence not found in nature, the protein has a combination of an RNA-binding domain and a DYW domain not found in nature, or a part not found in natural plant DYW proteins, e.g., nuclear localization signal sequence and human mitochondrial localization signal sequence, is added to a protein for RNA editing in animal cells. Nuclear transition signal sequences include, for example, PKKKRKV (SEQ ID NO: 32) derived from the SV40 large T antigen, and KRPAATKKAGQAKKKK (SEQ ID NO: 33), which is NLS of nucleoplasmin.

The number of PPR motifs can be appropriately determined depending on the sequence of the target RNA. The number of PPR motifs can be at least one, and can be two or more. It is known that two PPR motifs can bind to RNA (Nucleic Acids Research, 2012, Vol. 40, No. 6, 2712-2723).

One of the preferred embodiments of the DYW protein is the following:
an artificial DYW protein for editing a target RNA comprising an RNA-binding domain that contains at least one, preferably 2 to 25, more preferably 5 to 20, further preferably 10 to 18, of PPR motifs, and can sequence-specifically bind to a target RNA according to the rules of the PPR codes, and a DYW domain that is any one of DYW:PG, DYW:WW, and DYW: KP mentioned above.

Another one of the preferred embodiments of the DYW protein is the following:
a DYW protein for editing a target RNA comprising an RNA-binding domain that contains at least one, preferably 2 to 25, more preferably 5 to 20, further preferably 10 to 18, of PPR motifs, and can sequence-specifically bind to an animal target RNA according to the rules of the PPR codes (preferably, an RNA-binding domain that is a PLS type PPR protein), and a DYW domain that is any one of DYW:PG, DYW:WW, and DYW: KP mentioned above.

The DYW domain, PPR protein as the RNA-binding domain, and DYW protein of the present invention can be prepared by methods well known to those skilled in the art even in a relatively large amount. Such methods may include determining a nucleic acid sequence encoding an objective domain or protein from the amino acid sequence of the objective domain or protein, cloning it, and producing a transformant that produces the objective domain or protein.

### [Use of DYW protein]

### (Nucleic acid encoding DYW protein etc., vector, and cell)

The present invention also provides a nucleic acid encoding the PPR motif, or DYW protein, and a vector containing such a nucleic acid (e.g., vector for amplification, and expression vector). The vector may be a virus vector. For the vector for amplification, *E*. *coli* or yeast may be used as the host. In this description, expression vector means a vector containing, for example, a DNA having a promoter sequence, DNA encoding a desired protein, and DNA having a terminator sequence from the upstream side, but they need not necessarily be arranged in this order, so long as the desired function is exerted. In the present invention, recombinant vectors prepared by using various vectors that may be normally used by those skilled in the art may be used.

Specifically, the present invention provides a nucleotide sequence encoding a DYW protein comprising an RNA-binding domain (preferably an RNA-binding domain that is a PLS-type PPR protein) that contains at least one PPR motif and can sequence-specifically bind to a target RNA (preferably an animal target RNA) according to the rules of the PPR codes, and a DYW domain that is any one of DYW:PG, DYW:WW, and DYW:KP mentioned above.

Specifically, the present invention also provides a vector for editing a target RNA containing a nucleotide sequence encoding a DYW protein comprising an RNA-binding domain (preferably an RNA-binding domain that is a PLS-type PPR protein) that contains at least one PPR motif and can sequence-specifically bind to a target RNA (preferably an animal target RNA) according to the rules of the PPR codes, and a DYW domain that is any one of DYW:PG, DYW:WW, and DYW:KP mentioned above.

The DYW protein of the present invention can function in eukaryotic (e.g., animal, plant, microbe (yeast, etc.), and protozoan) cells. The DYW protein of the present invention can function, in particular, in animal cells (in vitro or in vivo). Examples of animal cells into which the DYW protein of the present invention or a vector expressing it can be introduced include, for example, cells derived from human, monkey, pig, cow, horse, dog, cat, mouse, and rat. Examples of cultured cells into which the DYW protein of the present invention or a vector expressing it can be introduced include, for example, Chinese hamster ovary (CHO) cells, COS-1 cells, COS-7 cells, VERO (ATCC CCL-81) cells, BHK cells, canine kidney-derived MDCK cells, hamster AV-12-664 cells, HeLa cells, WI38 cells, HEK293 cells, HEK293T cells, and PER.C6 cells, but not limited to these.

### (Use)

By using the DYW protein of the present invention, an editing target C contained in a target RNA can be converted into U, or an editing target U to C. RNA-binding PPR proteins are involved in all the RNA processing steps found in organelles, i.e., cleavage, RNA editing, translation, splicing, RNA stabilization etc.

The DYW protein of this invention also enables mitochondrial RNA single nucleotide editing. Mitochondria have their own genomes, which encode the component proteins of important complexes involved in respiration and ATP production. It is known that their mutations cause various diseases. Mutation repair utilizing the present invention is expected to enable treatment of various diseases.

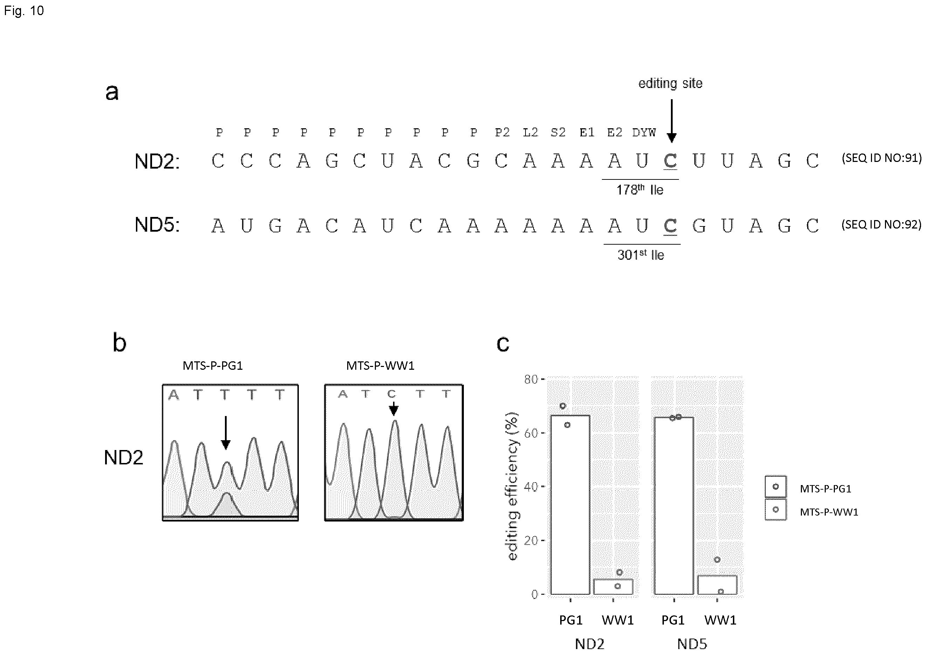

By the way, the CRISPR-Cas system has been developed as a C-to-U RNA editing tool in the cytoplasm by fusing the Cas protein to a modified ADAR domain (Abudayyeh et al., 2019). However, the CRISPR-Cas system is constituted by a protein and a guide RNA, and efficient mitochondrial transport of the guide RNA is difficult. In contrast, PPR proteins are capable of RNA editing as a single molecule, and in general, proteins can be delivered to mitochondria by fusing a mitochondria localization signal sequence to the N-terminal side of the proteins. Therefore, to confirm whether this technique can be used for mitochondrial RNA editing, PPR proteins targeting MT-ND2 and MT-ND5 were designed, and genes for such PPR proteins fused with the PG or WW domain were created (Fig. 10a).

These proteins consisting of a mitochondria targeting sequence (MTS) and PPR-P sequence target the third positions of 178th and 301st codons of MT-ND2 and MT-ND5 so as not to adversely affect HEK293T cells (Fig. 10a). After they were introduced into HEK293T cells using plasmids, editing was confirmed within mRNAs of MT-ND2 and MT-ND5 (Figs. 10b, and 10c). Up to 70% editing activity of these four proteins were detected for their targets, and no off-target mutation was detected for the same mRNA molecules (Figs. 10b, and 10c).

Therefore, such uses of the method for editing an RNA base provided by the present invention in a variety of fields as mentioned below can be expected.
(1) Medical care
   - Recognizing and editing a specific RNA relating to a specific disease. By utilizing the present invention, genetic diseases caused by single nucleotide mutations can be treated. The direction of most mutations in genetic diseases is from C to U. Therefore, the method of the present invention that can convert U to C can be especially useful.
   - Preparing cells with controlled repression and expression of RNA. Such cells include stem cells monitored for differentiation and undifferentiation states (e.g., iPS cells), model cells for evaluation of cosmetics, and cells of which expression of a functional RNA can be turned on/off for the purpose of elucidating drug discovery mechanisms and pharmacological testing.
(2) Agriculture, forestry and fisheries
   - Improvement of yield and quality of crops, forest products and marine products.
   - Breeding of organisms with improved disease resistance, improved environmental tolerance, or improved or new function.

For example, concerning hybrid first generation (Fl) plant crops, an Fl plant can be artificially created by mitochondrial RNA editing using a DYW protein, which may result in improvement of yield and quality of the crops. RNA editing using a DYW protein more accurately and quickly enable variety improvement and breeding (genetic improvement of organisms) of organisms compared with the conventional techniques. In addition, it can be said that RNA editing using a DYW protein is similar to the classical breeding methods such as selection of mutants and backcrossing, since they do not transform traits with a foreign gene as in genetic recombination. Therefore, they can also surely and quickly cope with global-scale food and environmental problems.

### (3) Chemistry

- Controlling protein expression amount in the production of useful substances using microorganisms, cultured cells, plant bodies, and animal bodies (e.g., insect bodies) by manipulating RNA. Productivity of useful substances can be thereby improved. Examples of the useful substances are proteinaceous substances such as antibodies, vaccines, and enzymes, as well as relatively low-molecular weight compounds such as pharmaceutical intermediates, fragrances, and dyes.
- Improvement of production efficiency of biofuel by modification of metabolic pathways of algae and microorganisms.

### [Terminology.]

Numerical value ranges represented as x to y include the values x and y at both ends of the ranges, unless especially stated.

Amino acid residue may be referred to simply as amino acid for amino acid sequences of proteins or polypeptides.

The term "identity" used for base sequence (also referred to as nucleotide sequence) or amino acid sequence means percentage of number of matched bases or amino acids shared between two sequences aligned in an optimal manner, unless especially stated. In other words, the identity can be calculated in accordance with the equation: Identity = (Number of matched positions/Total number of positions) x 100, and it can be calculated by using commercially available algorithms. Such algorithms are also incorporated in the NBLAST and XBLAST programs described in Altschul et al., J. Mol. Biol., 215 (1990) 403-410. In more detail, the search and analysis for the identity of nucleotide or amino acid sequences can be performed with algorithms or programs well known to those skilled in the art (e.g., BLASTN, BLASTP, BLASTX, and ClustalW). In the case of using a program, parameters can be appropriately set by those skilled in the art, or the default parameters of each program can also be used. The specific procedures of these analysis methods are also well known to those skilled in the art.

For nucleotide or amino acid sequences, a higher sequence identity is more preferred, unless especially noted. Specifically, it is preferably 40% or higher, more preferably 45% or higher, further preferably 50% or higher, further preferably 55% or higher, further preferably 60% or higher, further preferably 65% higher. It is also preferably 70% or higher, more preferably 80% or higher, further preferably 85% or higher, further preferably 90% or higher, further preferably 95% or higher, further preferably 97.5% or higher.

For polypeptides or proteins, number of amino acids in the expression of "sequence derived by substitution, deletion, or addition of amino acids" is not particularly limited in any motif or protein, so long as the motif or protein comprising the amino acid sequence has the desired function, unless especially stated. However, the number of amino acids to be substituted, or the like may be about 1 to 9 or 1 to 4, and if they are substituted with amino acids having similar properties, even larger number of amino acids may be substituted or the like. The means for preparing polynucleotides or proteins having such amino acid sequences are well known to those skilled in the art.

Amino acids having similar properties refer to amino acids with similar physical properties such as hydropathy, charge, pKa, and solubility, and refer to such amino acid as mentioned below, for example.
Hydrophobic (non-polar) amino acids; alanine, valine, glycine, isoleucine, leucine, phenylalanine, proline, tryptophan, tyrosine.
Non-hydrophobic amino acids; arginine, asparagine, aspartic acid, glutamic acid, glutamine, lysine, serine, threonine, cysteine, histidine, methionine.
Hydrophilic amino acids; arginine, asparagine, aspartic acid, glutamic acid, glutamine, lysine, serine, threonine.
Acidic amino acids: aspartic acid, glutamic acid.
Basic amino acids: lysine, arginine, histidine.
Neutral amino acids: alanine, asparagine, cysteine, glutamine, glycine, isoleucine, leucine, methionine, phenylalanine, proline, serine, threonine, tryptophan, tyrosine, valine.
Sulfur-containing amino acids: methionine, cysteine.
Aromatic ring-containing amino acids: tyrosine, tryptophan, phenylalanine.

### Examples

### [1. Design of DYW domain and measurement of RNA editing activity in E. coli] [Results]

### (Design of DYW domain)

Since most of the currently available plant genomic information is for angiosperms, the development of artificial DYW proteins is limited to those of the DYW:PG type. On the other hand, transcriptome data also include information on early land plant species showing both C-to-U and U-to-C RNA editing, although the transcriptome data are for partial sequences. Therefore, we designed completely artificial DYW domains using a database of PPR proteins constructed on the basis of transcriptome dataset created by the 1000 Plants (1KP) International Consortium (Non-patent document 11 mentioned above).

In designing the artificial DYW domains, in addition to the cytidine deaminase-like DYW domain, the PPR (P2) motifs and PPR-like (L2, S2, E1 and E2) motifs were also used. This is because the importance of these motifs for the RNA editing activity had been unknown at that time. We generated phylogenetic trees of the DYW domains of hornworts, lycophytes, and ferns, and classified them into three groups: DYW:PG, DYW:WW, and DYW:KP. Then, new phylogenetic trees were created for the DYW domains of each of these groups to select phylogenetic groups (clades) of proteins that could be used to design artificial DYW domains (Fig. 1). The DYW:PG group had large differences in sequences, making it difficult to select a clade of proteins used for the design (Fig. 1a). The protein sequences of lycophytes showed great diversity, the number of the proteins was also huge, and therefore we decided to focus on this protein group (PG1). For the design of the DYW:WW domain, we selected a clade of proteins with short branches found only in hornworts (Fig. 1b, WW1). The short branching of this clade suggests that genetic variation among proteins is small. Then, to design the DYW:KP domain, we focused on a clade of DYW domain specific to ferns. The protein sequences of this clade have significant amino acid mutations, but conserve the lengths thereof (Fig. 1c, KP1).

### (Design of RNA-binding domain)

We fused each DYW domain with an artificial P or PLS array designed on the basis of PPR motif sequences identified from plant genomic information (Non-patent document 2 mentioned above) (Fig. 2a). The PPR motif of the P array was constructed on the basis of a consensus sequence obtained by alignment of the 35 amino acids P motifs, and few amino acids were replaced in order to improve the RNA recognition. For the design of the PLS array, the standard length 35 amino acids motifs (P1 and L1) and the 31 amino acids motif (S1) were chosen. In natural PPR proteins, the first and last P1L1S1 triplets show clear differences in amino acid residues at specific positions, and are different from the internal P1L1S1 triplets. To design an artificial PLS array as close as possible to the naturally occurring one, three types of P1L1S1 were designed according to the positions of the motifs in the PPR proteins, i.e., the first P1L1S1 (N-terminal side), internal P1L1S1, and the last (C-terminal side) P1L1S1 located just before P2L2S2. Hereafter, these proteins are named on the basis of their structures. For example, the DYW:WW domain fused to the P array will be designated P-DYW:WW.

### (Artificial DYW proteins can specifically edit their target sequences)

In *Arabidopsis thaliana,* the PPR protein CLB19 recognizes RNA editing sites on chloroplast rpoA and clpP RNAs. We decided to design a PPR protein that targets the rpoA editing site. The fourth and ii-th amino acids in the P1, L1, S1, and P2 motifs were determined according to the PPR codes. On the other hand, since the PPR codes for the C-terminal PPR-like motifs (L2, S2, E1, and E2) were unknown, the fourth and ii-th amino acids of CLB19 were used for the L2, S2, E1 and E2 motifs (Fig. 2b).

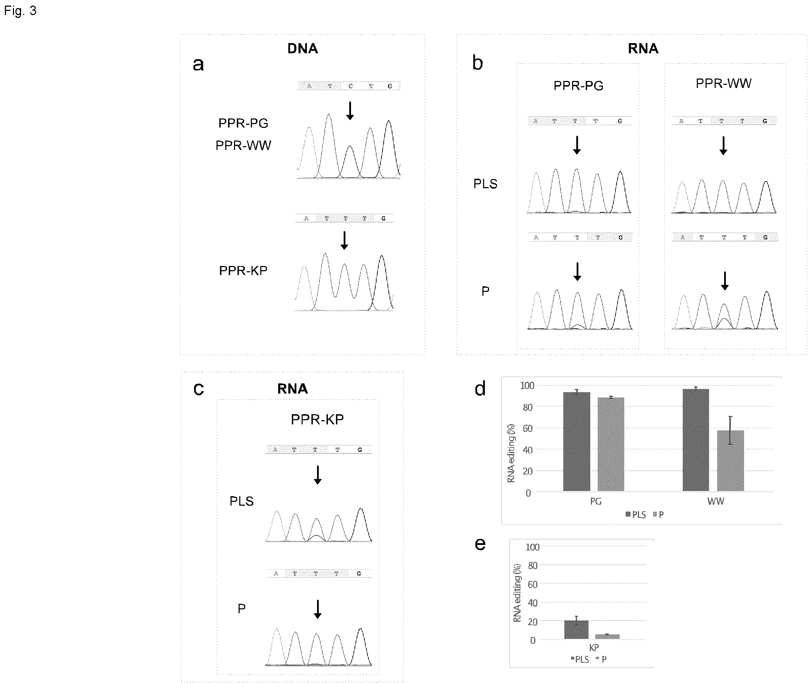

A gene region encoding a recombinant artificial DYW protein was cloned into an expression vector, and the target sequence was added downstream of the termination codon. On the basis of previous studies on two PPR proteins (PPR56 and PPR65) of *Physcomitrella patens* (Non-patent document 12 mentioned above), a method for assaying an RNA editing activity of a designed PPR protein in *E. coli* was developed. The designed PLS-DYW:PG1 and PLS-DYW:WW1 showed no editing activity against DNA, whereas they replaced cytidine with uridine for RNA with an editing efficiency higher than 90% (Figs. 3a, 3b, and 3d). When the P array was used instead of the PLS domain, the editing efficiency decreased by 10 to 40%. On the other hand, a U-to-C RNA editing activity was observed for both P-DYW:KP and PLS-DYW:KP (Figs. 3c, and 3e). However, their editing activities were lower than those of DYW:PG and DYW:WW.

### [Methods]

### (Phylogenetic tree)

The P2-L2-S2-E1-E2-DYW regions including the DYW domain (minimum length is 132 amino acids) were extracted from the PPR database (https://ppr.plantenergy.uwa.edu.au/onekp/, Non-patent document 11 mentioned above). Alignment of the obtained sequences was created by using MAFFT L-INS-i (v7.407 automatic mode) (K. Katoh, D.M. Standley (2013) Mol. Biol. Evol., 30(4):772-780), and then trimmed by using trimAl (v.1.4.rev15) (Salvador Capella-Gutierrez, et al. (2009) Bioinformatics, 25(15):1972-1973). For the trimming, the parameters were set to gt 0.2 cons 20. Sequences having mutations in the active site (HxExₙCxxCH) were excluded from the alignment (Fig. 4).

Phylogenetic trees were created from the remaining sequences by using FastTree (v2.1.10) (Price, M. N., et al. (2010) PLoS One 5:e9490), and DYW:PG, DYW:WW and DYW:KP were identified. The parameters used were wag and cat 8.

### (Cloning of Trx-PPR-DYW proteins and target sequences)

The consensus sequences of the DYW domains (including P2, L2, S2, E1, and E2) were designed by using EMBOSS: cons (v.6.6.0.0). As the vector for protein expression, pET21b+PA was modified by removing the original Esp3I and Bpil restriction enzyme sites and adding two Esp3I sites as cloning sites. The gene was constructed in four sections (Trx, PPR array, DYW domain, and RNA editing site) by using the two-step Golden Gate assembly method. First, into the Esp3I site of the modified pET21b vector, 1) the thioredoxin-oxHis-TEV gene region (including a Bpil restriction enzyme site at 3'), 2) the P2-L2-S2-E1-E2-DYW gene region (including a Bpil restriction enzyme site at 5'), and 3) the coding sequence region of the RNA editing site were cloned. Then, the full-length PLS or P domain was cloned into the Bpil site to produce PLS:DYW (SEQ ID NOS: 35 to 37) or P:DYW (SEQ ID NOS: 38 to 40) proteins.

### (Measurement of RNA editing activity in E. coli)

For analysis of the RNA editing activity of the recombinant proteins in *E. coli,* we modified the protocol developed by Oldenkott et al. (Non-patent document 12 mentioned above). The plasmid DNAs prepared above were introduced into *E. coli* Rosetta 2 strain, and the cells were incubated overnight at 37°C in 1 mL of LB medium (containing 50 µg/mL of carbenicillin and 17 µg/mL of chloramphenicol). LB medium containing appropriate antibiotics was prepared in a volume of 5 mL on a deep-bottom 24-well plate, and 100 µL of the preculture was inoculated into the medium. The cells were grown at 37°C and 200 rpm until the absorbance (OD₆₀₀) reached 0.4 to 0.6, and then the plate was cooled at 4°C for 10 to 15 minutes. Then, 0.4 mM ZnSO₄ and 0.4 mM IPTG were added, and the culture was further continued at 16°C and 180 rpm for 18 hours. After collecting 750 µL of the culture medium and centrifuging it, the bacterial pellet was frozen in liquid nitrogen and stored at -80°C.

The frozen cell pellet was resuspended in 200 µL of 1-thioglycerol homogenized solution, and sonicated at 40 W for 10 seconds to separate the cells, and 200 µL of lysis buffer was added. RNA was extracted by using Maxwell (registered trademark) RSC simplyRNA Tissue Kit (Promega). RNA was treated with DNaseI (Takara Bio), and cDNA was synthesized by using SuperScript (registered trademark)

III Reverse Transcriptase (Invitrogen) with 1 µg of the treated RNA and 1.25 µM random primers (6-mer). NEBNext High-Fidelity 2x PCR Master Mix (New England Biolabs), 1 µL of cDNA, and primers for the thioredoxin and T7 terminator sequences were used to amplify the region including the editing site. The PCR product was purified by using NucleoSpin (registered trademark) Gel and PCR Cleanup (Takara Bio), and sequencing analysis was performed by using forward primers specific to the DYW domain sequence to determine the bases of the RNA editing site. RNA editing efficiency was calculated by using a ratio of heights of waveform peaks of C and U in the editing site in accordance with the equation, U/(C + U) × 100 for the C-to-U editing efficiency, or C/(C + U) × 100 for the U-to-C editing efficiency. Independent experiments were repeated three times.

### [List of Cited Sequences]

**[Table 3-1]**

| PLS array (3 P1L1S1 triplets) | | SEQ ID NO: |
|---|---|---|
| P1 | FSWNSMIRGYARSGQPEEALSLFSQMRRSGIKPDS | 4 |
| L1 | YTFPFVLKACASLSSLEEGKQIHAHVIKSGFESDV | 5 |
| S1 | YVQSSLIDMYAKCGSLEDARKVFDEMPERNV | 6 |
| P1 | VSWNAMISGYAQNGQSEEALELFREMQKEGIKPSE | 7 |
| L1 | FTFCSVLKACASLGSLEMGKQIHGYVIKSGFESIV | 8 |
| S1 | FVGNALIDMYAKCGSLEDARKVFDEMPERTV | 9 |
| P1 | VSWTAMISGYAQNGQSEEALELFREMQREGVKPDE | 10 |
| L1 | VTLPSVLSACANLGALEQGKQIHAYVIKNGFESDV | 11 |
| S1 | FVGSALIDMYAKCGSIEEARKVFDEMPEKDV | 12 |

| P array (10 PPR motifs) | | SEQ ID NO: |
|---|---|---|
| P | VTYTTLIDGLCKAGKVDEALELFKEMRSKGVKPNV | 13 |
| P | VTYNTLIDGLCKAGRLDEAEELLEEMEEKGIKPDV | 14 |
| P | VTYNTLIDGLCKAGRLDEAEELLEEMEEKGIKPDV | 14 |
| P | VTYTTLIDGLCKAGKVDEALELFKEMRSKGVKPNV | 13 |
| P | VTYNTLIDGLCKSGKIEEALKLFKEMEEKGITPSV | 15 |
| P | VTYTTLIDGLCKAGKVDEALELFKEMRSKGVKPNV | 13 |
| P | VTYNTLIDGLCKSGKIEEALKLFKEMEEKGITPSV | 15 |
| P | VTYTTLIDGLCKAGKVDEALELFDEMKERGIKPDV | 16 |
| P | VTYNTLIDGLCKAGRLDEAEELLEEMEEKGIKPDV | 14 |
| P | VTYTTLIDGLCKAGKVDEALELFDEMKERGIKPDV | 16 |

**[Table 3-2]**

| DYW:PG | | SEQ ID NO: |
|---|---|---|
| P2 | VSWNAMIAAYAQHGHGKEALQLFQQMQQEGVKPS E | 17 |
| L2 | VTFTSILSACSHAGLVDEGHHYFESMSPDYGITPRV | 18 |
| S2 | EHYGCMVDLLGRAGRLDEAEDLIKSMPFQPNV | 19 |
| E1 | VVWGTLLGACRVHGDVERGERAAERILELDPESA | 20 |
| E2 | APYVLLSNIYAAAGRWDEAAKVRKLMKERGVKK | 21 |
| DYW | EPGCSWIEVNNKVHEFVAGDKSHPQTKEIYAELERLS KQMKEAGYVPDTKFVLHDVEEEEKEQLLCYHSEKL AIAFGLISTPPGTPLRIIKNLRVCGDCHTATKFISKIVG | 1 |
| | REIVVRDANRFHHFKDGVCSCGDYW | |

| DYW:WW | | SEQ ID NO: |
|---|---|---|
| P2 | VTWNALIAGYARQGESDLVFHLLERMRQEGIQPSG | 22 |
| L2 | VTFTSVLTVCSHAGLVDEGQKYFDAMSEDYGITPRI | 23 |
| S2 | EHYGCMVDLLGRAGQMDEAVAMVEKMPFQPNL | 24 |
| E1 | VTWGTLLGACRKWNNVEIGRHAFECAVRLDEKSA | 25 |
| E2 | AAYVLMSNIYADAHMWEERDKIQAMRKNARAWK | 26 |
| DYW | KPGQSWWTDTGGIVHTFVVGDTKHPQSQDIYAKLK DLYVKMKEEGYVPHLDCVLWDISDDEKEDALCGHS EKLAIACALINTPPGTPIRIVKNLRVCDDCHKAIALIS KIEGRNIICRDASRFHNYKDGKCSCGDYW | 2 |

| DYW:KP | | SEQ ID NO: |
|---|---|---|
| P2 | VAWNSMIKGYGMNHEGKMAVQCFEDMQKQGVKPS A | 27 |
| L2 | VTFTCLLTACSHASLVSEGQEYFKMMREEYGIAPRV | 28 |
| S2 | EHYGCMVDLLARSGHLYEAEKFLEMLCPPNE | 29 |
| E1 | GTWGALLSACKTYGEVELGLRCFQQLVQLNPESA | 30 |
| E2 | AWYVLMADIYAGAGRWDDAYRIEELRKHAGAKK | 31 |
| DYW | KPASALIEVNKKVHEFVVGNNQSEEISAMLKSLNSR MKEEGHVPNLDLVLKPVSDEEKEAALCEHAEKLAIA FGLLNTPQGQTLRVTKNLRMCNDCHNASKIISKIERR EIILRDDCCIHHFKDGLCSCGDMF | 3 |

**[Table 3-3]**

| |
|---|
| <PLS array (3 P1L1S1 triplets) + DYW:PG-lyc> |
| VFSWNSMIRGYARNGQPEEALSLYSQMRRSGIKPDNYTFPFVLKACASLSSLKEGKQIHGHVIKSGFESDVYVQSALIDMYAKCGE LEDARKVFDEMPERNVVSWNAMISGYAQNGQSEEALELFREMQQEGIKPSEFTFCSVLSACASLGSLEMGKQIHGYVIKSGFESIV FVGNALIDMYAKCGSIEDARKVFDEMPERTVVSWTAMISGYAQNGQSEEALELFREMQREGVKPDEVTLPSVLSACANLGALEQGK QIHAYVIKSGFESDVFVGSALIDMYAKCGSIEDARKVFDKMPERDVVSWNAMIAAYAQHGHGKEALQLFQQMQQEGVKPSEVTFTS ILSACSHAGLVDEGHHYFESMSPDYGITPRVEHYGCMVDLLGRAGRLDEAEDLIKSMPFQPNVVVWGTLLGACRVHGDVERGERAA ERILELDPESAAPYVLLSNIYAAAGRWDEAAKVRKLMKERGVKKEPGCSWIEVNNKVHEFVAGDKSHPQTKEIYAELERLSKQMKE AGYVPDTKFVLHDVEEEEKEQLLCYHSEKLAIAFGLISTPPGTPLRIIKNLRVCGDCHTATKFISKIVGREIVVRDANRFHHFKDG VCSCGDYW |
| (SEQ ID NO:35) |
| <PLS array (3 P1L1S1 triplets) + DYW:WW-WWS> |
| VFSWNSMIRGYARNGQPEEALSLYSQMRRSGIKPDNYTFPFVLKACASLSSLKEGKQIHGHVIKSGFESDVYVQSALIDMYAKCGE LEDARKVFDEMPERNVVSWNAMISGYAQNGQSEEALELFREMQQEGIKPSEFTFCSVLSACASLGSLEMGKQIHGYVIKSGFESIV FVGNALIDMYAKCGSIEDARKVFDEMPERTVVSWTAMISGYAQNGQSEEALELFREMQREGVKPDEVTLPSVLSACANLGALEQGK QIHAYVIKSGFESDVFVGSALIDMYAKCGSIEDARKVFDKMPERDVVTWNALIAGYARQGESDLVFHLLERMRQEGIQPSGVTFTS VLTVCSHAGLVDEGQKYFDAMSEDYGITPRIEHYGCMVDLLGRAGQMDEAVAMVEKMPFQPNLVTWGTLLGACRKWNNVEIGRHAF ECAVRLDEKSAAAYVLMSNIYADAHMWEERDKIQAMRKNARAWKKPGQSWWTDTGGIVHTFVVGDTKHPQSQDIYAKLKDLYVKMK EEGYVPHLDCVLWDISDDEKEDALCGHSEKLAIACALINTPPGTPIRIVKNLRVCDDCHKAIALISKIEGRNIICRDASRFHNYKD GKCSCGDYW |
| (SEQ ID NO:36) |
| <PLS array (3 P1L1S1 triplets) + DYW:KP-lepto> |
| VFSWNSMIRGYARNGQPEEALSLYSQMRRSGIKPDNYTFPFVLKACASLSSLKEGKQIHGHVIKSGFESDVYVQSALIDMYAKCGE LEDARKVFDEMPERNVVSWNAMISGYAQNGQSEEALELFREMQQEGIKPSEFTFCSVLSACASLGSLEMGKQIHGYVIKSGFESIV FVGNALIDMYAKCGSIEDARKVFDEMPERTVVSWTAMISGYAQNGQSEEALELFREMQREGVKPDEVTLPSVLSACANLGALEQGK QIHAYVIKSGFESDVFVGSALIDMYAKCGSIEDARKVFDKMPERDVVAWNSMIKGYGMNHEGKMAVQCFEDMQKQGVKPSAVTFTC LLTACSHASLVSEGQEYFKMMREEYGIAPRVEHYGCMVDLLARSGHLYEAEKFLEMLCPPNEGTWGALLSACKTYGEVELGLRCFQ QLVQLNPESAAWYVLMADIYAGAGRWDDAYRIEELRKHAGAKKKPASALIEVNKKVHEFVVGNNQSEEISAMLKSLNSRMKEEGHV PNLDLVLKPVSDEEKEAALCEHAEKLAIAFGLLNTPQGQTLRVTKNLRMCNDCHNASKIISKIERREIILRDDCCIHHFKDGLCSC GDMF |
| (SEQ ID NO:37) |
| <P array (10 PPR motifs) + DYW:PG-lyc> |
| VVTYTTLIDGLCKAGKVDEALELFKEMRSKGVKPNVVTYNTLIDGLCKAGRLDEAEELLEEMEEKGIKPDVVTYNTLIDGLCKAGR LDEAEELLEEMEEKGIKPDVVTYTTLIDGLCKAGKVDEALELFKEMRSKGVKPNVVTYNTLIDGLCKSGKIEEALKLFKEMEEKGI TPSVVTYTTLIDGLCKAGKVDEALELFKEMRSKGVKPNVVTYNTLIDGLCKSGKIEEALKLFKEMEEKGITPSVVTYTTLIDGLCK AGKVDEALELFDEMKERGIKPDVVTYNTLIDGLCKAGRLDEAEELLEEMEEKGIKPDVVTYTTLIDGLCKAGKVDEALELFDEMKE RGIKPDVVSWNAMIAAYAQHGHGKEALQLFQQMQQEGVKPSEVTFTSILSACSHAGLVDEGHHYFESMSPDYGITPRVEHYGCMVD LLGRAGRLDEAEDLIKSMPFQPNVVVWGTLLGACRVHGDVERGERAAERILELDPESAAPYVLLSNIYAAAGRWDEAAKVRKLMKE RGVKKEPGCSWIEVNNKVHEFVAGDKSHPQTKEIYAELERLSKQMKEAGYVPDTKFVLHDVEEEEKEQLLCYHSEKLAIAFGLIST PPGTPLRIIKNLRVCGDCHTATKFISKIVGREIVVRDANRFHHFKDGVCSCGDYW |
| (SEQ ID NO:38) |
| <P array (10 PPR motifs) + DYW:WW-WW5> |
| VVTYTTLIDGLCKAGKVDEALELFKEMRSKGVKPNVVTYNTLIDGLCKAGRLDEAEELLEEMEEKGIKPDVVTYNTLIDGLCKAGR LDEAEELLEEMEEKGIKPDVVTYTTLIDGLCKAGKVDEALELFKEMRSKGVKPNVVTYNTLIDGLCKSGKIEEALKLFKEMEEKGI TPSVVTYTTLIDGLCKAGKVDEALELFKEMRSKGVKPNVVTYNTLIDGLCKSGKIEEALKLFKEMEEKGITPSVVTYTTLIDGLCK AGKVDEALELFDEMKERGIKPDVVTYNTLIDGLCKAGRLDEAEELLEEMEEKGIKPDVVTYTTLIDGLCKAGKVDEALELFDEMKE RGIKPDVVTWNALIAGYARQGESDLVFHLLERMRQEGIQPSGVTFTSVLTVCSHAGLVDEGQKYFDAMSEDYGITPRIEHYGCMVD LLGRAGQMDEAVAMVEKMPFQPNLVTWGTLLGACRKWNNVEIGRHAFECAVRLDEKSAAAYVLMSNIYADAHMWEERDKIQAMRKN ARAWKKPGQSWWTDTGGIVHTFVVGDTKHPQSQDIYAKLKDLYVKMKEEGYVPHLDCVLWDISDDEKEDALCGHSEKLAIACALIN TPPGTPIRIVKNLRVCDDCHKAIALISKIEGRNIICRDASRFHNYKDGKCSCGDYW (SEQ ID NO:60) |
| (SEQ ID NO:39) |
| <P array (10 PPR motifs) + DYW:KP-lepto> |
| VVTYTTLIDGLCKAGKVDEALELFKEMRSKGVKPNVVTYNTLIDGLCKAGRLDEAEELLEEMEEKGIKPDVVTYNTLIDGLCKAGR LDEAEELLEEMEEKGIKPDVVTYTTLIDGLCKAGKVDEALELFKEMRSKGVKPNVVTYNTLIDGLCKSGKIEEALKLFKEMEEKGI TPSVVTYTTLIDGLCKAGKVDEALELFKEMRSKGVKPNVVTYNTLIDGLCKSGKIEEALKLFKEMEEKGITPSVVTYTTLIDGLCK AGKVDEALELFDEMKERGIKPDVVTYNTLIDGLCKAGRLDEAEELLEEMEEKGIKPDVVTYTTLIDGLCKAGKVDEALELFDEMKE RGIKPDVVAWNSMIKGYGMNHEGKMAVQCFEDMQKQGVKPSAVTFTCLLTACSHASLVSEGQEYFKMMREEYGIAPRVEHYGCMVD LLARSGHLYEAEKFLEMLCPPNEGTWGALLSACKTYGEVELGLRCFQQLVQLNPESAAWYVLMADIYAGAGRWDDAYRIEELRKHA GAKKKPASALIEVNKKVHEFVVGNNQSEEISAMLKSLNSRMKEEGHVPNLDLVLKPVSDEEKEAALCEHAEKLAIAFGLLNTPQGQ TLRVTKNLRMCNDCHNASKIISKIERREIILRDDCCIHHFKDGLCSCGDMF |
| (SEQ ID NO:40) |

### [2. Example in cultured animal cells]

### [Results]

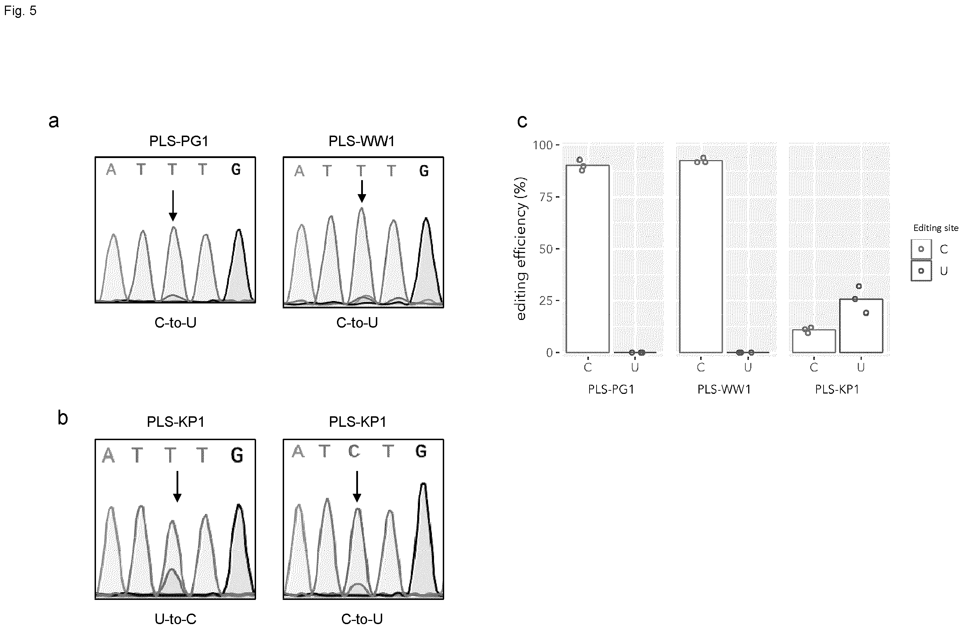

A plasmid containing a gene obtained by fusing genes for PLS-type PPR and each of the DYW domains (PG1 (SEQ ID NO: 1), WW1 (SEQ ID NO: 2), and KP1 (SEQ ID NO: 3)), and the target sequence was transfected into HEK293T cells, the cells were cultured, and then RNA was collected. The conversion efficiency of cytidine (C) to uridine (U) or uridine (U) to cytidine (C) at the target site was analyzed by Sanger sequencing (Fig. 5). When PG1 or WW1 domain was fused, C-to-U activity was observed at an efficiency of 90% or higher, while no U-to-C activity was detected (Figs. 5a, and 5c). When the KP1 domain was fused, U-to-C activity was detected at an efficiency of 25%, and C-to-U activity was also detected at an efficiency of about 10% (Figs. 5b and 5c). These results indicate that the editing enzymes function also in cultured animal cells.

### [Methods]

### (Preparation of PPR expression plasmid for animal cultured cell test)

From the plasmid used in the assay shown in Fig. 3, the region containing the 6xHis-PPR-DYW protein (the same protein as used in the experiment with *E. coli* (SEQ ID NOS: 35 to 37)) gene sequences and the editing site (SEQ ID NO: 34) was amplified by PCR, and cloned into an animal cell expression vector by the Golden Gate Assembly method. The vector expresses PPR under control by the CMV promoter and a promoter including human β-globin chimeric intron, and a poly A signal is added by the SV40 polyadenylation signal.

### (Culture of HEK293T cells)

The HEK293T cells were cultured in Dulbecco's Modified Eagle Medium (DMEM) containing high content glucose, glutamine, Phenol Red, and sodium pyruvate (Fujifilm Wako Pure Chemicals) and supplemented with 10% fetal bovine serum (Capricom) and 1 % penicillin-streptomycin (Fujifilm Wako Pure Chemicals) at 37°C and 5% CO₂. The cells were subcultured every 2 or 3 days when they reached 80 to 90% confluence.

### (Transfection)

For the RNA editing assay, approximately 8.0 ×10⁴ HEK293T cells were placed in each well of a 24-well flat-bottom cell culture plate, and cultured at 37°C and 5% CO₂ for 24 hours. To each well, 500 ng of the plasmid was added together with 18.5 µl of Opti-MEM (registered trademark) I Reduced Serum Medium (Thermo Fisher) and 1.5 µl of FuGENE (registered trademark) HD Transfection Reagent (Promega) to a final volume of 25 µl. The mixture was incubated at room temperature for 10 minutes before addition to the cells. The cells were collected after 24 hours from the transfection.

### (RNA extraction, reverse transcription, and sequencing)

These operations were performed in the same manners as those used in the aforementioned assay performed with *E. coli.* In the following examples, the experiments were performed in the same manners as those of the assay performed in *E. coli* and this example, unless especially noted.

### [3. Effect of domain swapping on RNA editing activity]

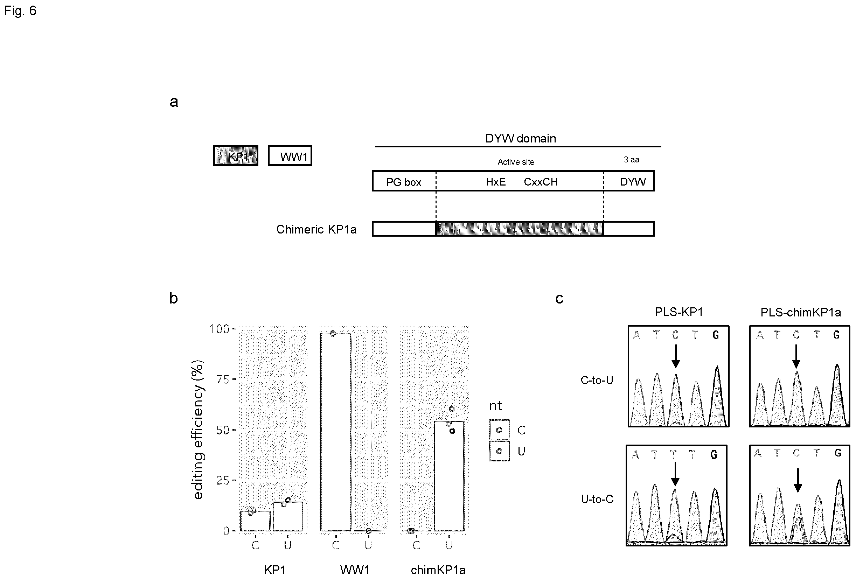

The DYW domain can be divided into several regions on the basis of conservation of amino acid sequence, but their relevance to the RNA editing activity had been unknown. Here, parts of the KP1 and WW1 domains were swapped, and effect of the swapping on the RNA editing activity was examined in HEK239T cells (Fig. 6). It was found that the protein consisting of the PG box and DYW of the WW1 domain fused to the center region of the KP1 domain containing the active site (chimKP1a, SEQ ID NO: 90) showed a U-to-C activity higher by almost 50% than that of the KP1 domain, and substantially lost the C-to-U activity. The U-to-C editing performance of the KP domain was successfully improved by the domain swapping.

### [4. Improvement of RNA editing activity of KP domain by mutagenesis]

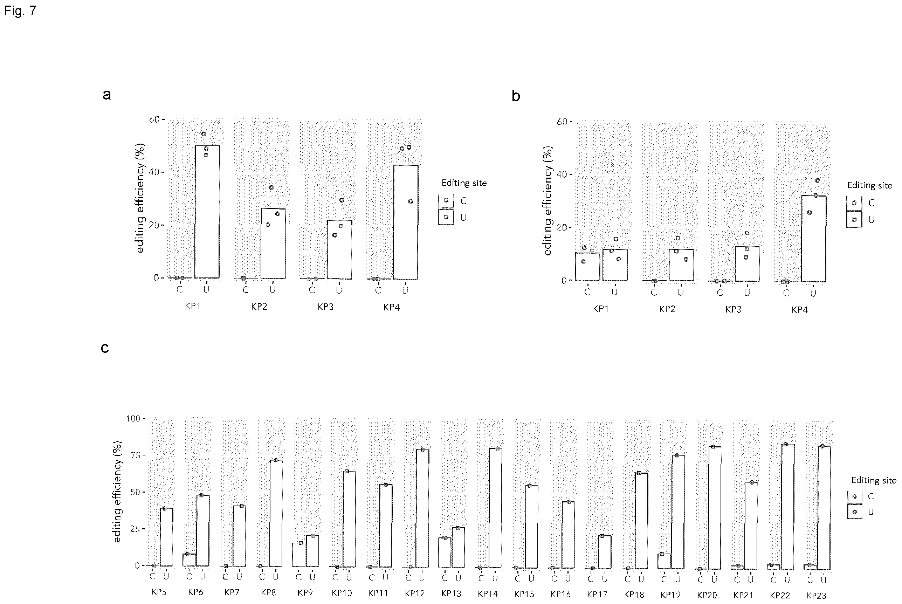

Various mutations were introduced into the KP domain to improve the RNA editing activity of the KP domain. KP2 to KP23 (SEQ ID NOS: 68 to 89) were designed, and their C-to-U or U-to-C RNA editing activities were examined in *E. coli* (Fig. 7a) and HEK293T cells (Figs. 7b, and 7c). KP22 (SEQ ID NO: 88) showed the highest U-to-C editing activity, and low C-to-U editing activity, and thus the RNA editing activity thereof was successfully improved compared with KP1.

### [5. Improvement of RNA editing activity of PG domain by mutagenesis]

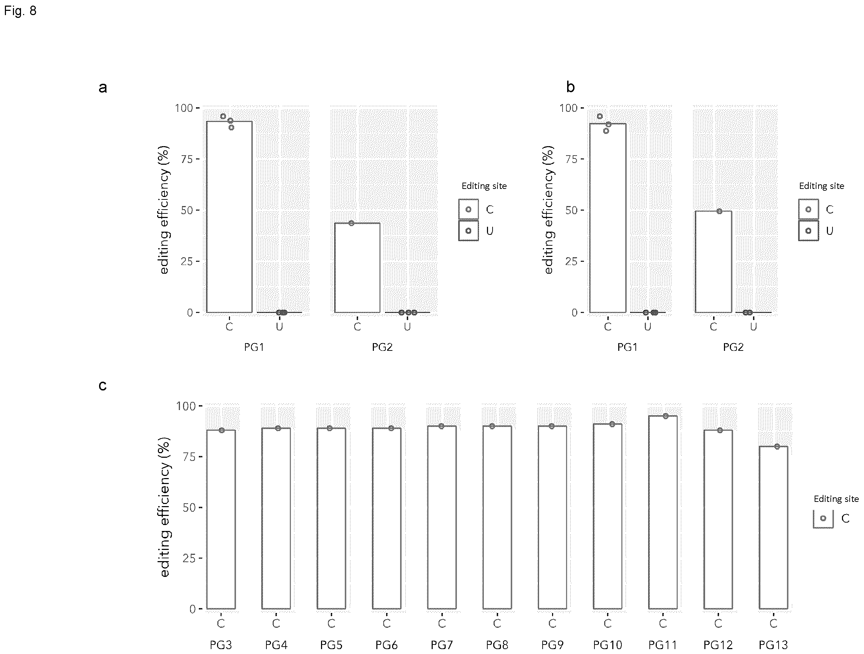

Various mutations were introduced into the PG domain to improve the RNA editing activity of the PG domain. PG2 to PG13 (SEQ ID NOS: 41 to 53) were designed, and their C-to-U RNA editing activities were examined in *E. coli* (Fig. 8a) and HEK293T cells (Figs. 8b, and 8c). PG11 (SEQ ID NO: 50) showed the highest C-to-U editing activity, and thus the RNA editing activity thereof was successfully improved.

### [6. Improvement of RNA editing activity of WW domain by mutagenesis]

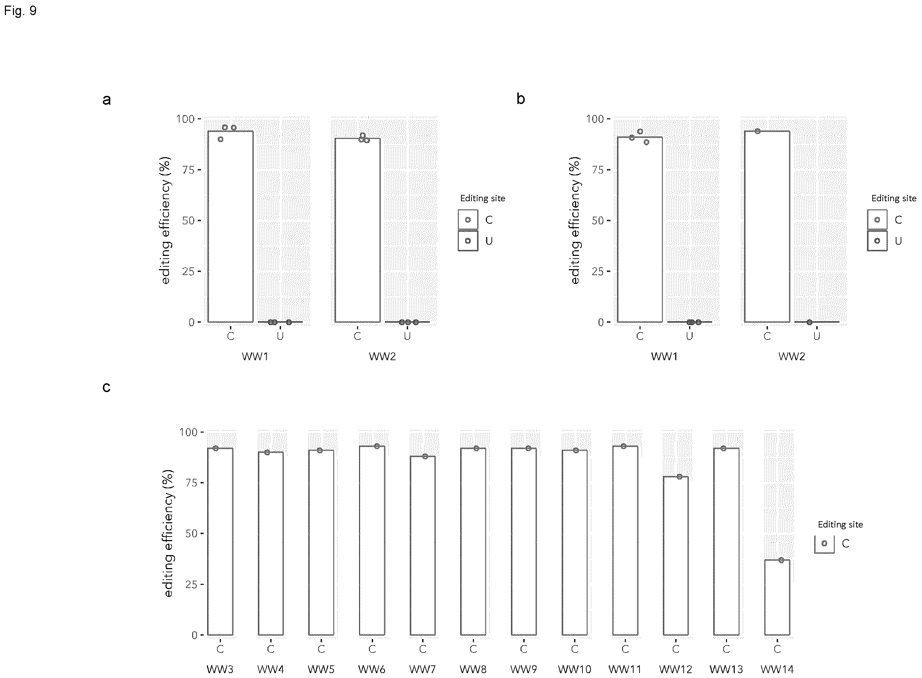

Various mutations were introduced into the WW domain to improve the RNA editing activity of the WW domain. WW2 to WW14 were designed, and their C-to-U RNA editing activities were examined in *E. coli* (Fig. 9a) and HEK293T cells (Figs. 9b, and 9c). WW11 (SEQ ID NO: 63) showed the highest C-to-U editing activity, and thus the RNA editing activity thereof was successfully improved compared with WW1.

### [7. Human mitochondria RNA editing using PPR protein]

### [Results]

Mitochondria have their own genomes, which encode the component proteins of important complexes involved in respiration and ATP production. It is known that mutations in them cause various diseases, and methods for repairing mutations are desired.

The CRISPR-Cas system has been developed as a tool for C-to-U RNA editing in the cytoplasm by fusing a modified ADAR domain to the Cas protein (Abudayyeh et al., 2019, Science, Vol. 365, Issue 6451, pp.382-386). However, the CRISPR-Cas system is constituted by a protein and guide RNA, and efficient mitochondrial transport of guide RNA is difficult. On the other hand, PPR proteins are capable of RNA editing as a single molecule, and in general, proteins can be delivered to mitochondria by fusing a mitochondria localization signal sequence to the N-terminal side thereof. Therefore, to confirm whether this technique can be used for mitochondria RNA editing, PPRs targeting MT-ND2 and MT-ND5 were designed, and genes for the proteins fused with the PG1 or WW1 domain were created (Fig. 10a).

These proteins consisting of a mitochondria targeting sequence (MTS) and P-DYW sequence target the third positions of the 178th and 301st codons of MT-ND2 and MT-ND5 so as not to adversely affect the HEK293T cells (Fig. 10a). After the genes were introduced into the HEK293T cells as plasmids, editing was confirmed in MT-ND2 and MT-ND5 mRNA (Figs. 10b, and 10c). For these four kinds of proteins, editing activities at an efficiency of up to 70% were detected for their targets, and no off-target mutations was detected for the same mRNA molecules (Fig. 10b, and 10c).

### [Methods]

### (Cloning for mitochondria RNA editing)

A mitochondria target sequence derived from LOC100282174 protein of Zea mays (Chin et al., 2018), 10 PPR-P and PPR-like motifs, and DYW domain moiety (PG1 and W W 1 were used as the DYW domain) were cloned into an expression plasmid under the control of the CMV promoter by the Golden Gate Assembly method (SEQ ID NOS: 91 to 94).

### [List of cited sequences]

**[Table 4-1]**

| | Sequence | SEQ ID NO: |
|---|---|---|
| PG1 | EPGCSWIEVNNKVHEFVAGDKSHPQTKEIYAELERLSKQMKEAGYVPDTKFVLHDVEEEEKEQLLCYHSE KLAIAFGLISTPPGTPLRIIKNLRVCGDCHTATKFISKIVGREIVVRDANRFHHFKDGVCSCGDYW | 1 |
| PG2 | | 41 |
| PG3 | | 42 |
| PG4 | | 43 |
| PG5 | | 44 |
| PG6 | | 45 |
| PG7 | | 46 |
| PG8 | | 47 |
| PG9 | | 48 |
| PG10 | | 49 |
| PG11 | | 50 |
| PG12 | | 51 |
| PG13 | | 52 |
| PG14 | | 53 |

**[Table 4-2]**

| | Sequence | SEQ ID NO: |
|---|---|---|
| WW1 | KPGQSWWTDTGGIVHTFVVGDTKHPQSQDIYAKLKDLYVKMKEEGYVPHLDCVLWDISDDEKEDALCGHS EKLAIACALINTPPGTPIRIVKNLRVCDDCHKAIALISKIEGRNIICRDASRFHNYKDGKCSCGDYW | 2 |
| WW2 | QPGQSWWTDMGGVVHTFVVGDREHPESQDIYAKLKDLIVKMKKEGYVPHLDSVLRDIPDDEKEDALCGHS EKLAIAYALLKTPEGTTIRVVKNLRVCEDCHTATALISKIEQRTIICRDASRFHVYKDGKCSCGDYW | 54 |
| WW3 | EPGQSWWTDTGGIVHTFVVGDTKHPQSQDIYAKLKDLYVKMKEEGYVPHLDCVLWDISDDEKEDALCGHS EKLAIACALINTPPGTPIRIVKNLRVCDDCHKAIALISKIEGRNIICRDASRFHNYKDGKCSCGDYW | 55 |
| WW4 | KPAQSWWTDTGGIVHTFVVGDTKHPQSQDIYAKLKDLYVKMKEEGYVPHLDCVLWDISDDEKEDALCGHS EKLAIACALINTPPGTPIRIVKNLRVCDDCHKAIALISKIEGRNIICRDASRFHNYKDGKCSCGDYW | 56 |
| WW5 | KPGQAWWTDTGGIVHTFVVGDTKHPQSQDIYAKLKDLYVKMKEEGYVPHLDCVLWDISDDEKEDALCGHS EKLAIACALINTPPGTPIRIVKNLRVCDDCHKAIALISKIEGRNIICRDASRFHNYKDGKCSCGDYW | 57 |
| WW6 | KPGQSTWTDTGGIVHTFVVGDTKHPQSQDIYAKLKDLYVKMKEEGYVPHLDCVLWDISDDEKEDALCGHS EKLAIACALINTPPGTPIRIVKNLRVCDDCHKAIALISKIEGRNIICRDASRFHNYKDGKCSCGDYW | 58 |
| WW7 | KPGQSWTDTGGIVHTFVVGDTKHPQSQDIYAKLKDLYVKMKEEGYVPHLDCVLWDISDDEKEDALCGHSE KLAIACALINTPPGTPIRIVKNLRVCDDCHKAIALISKIEGRNIICRDASRFHNYKDGKCSCGDYW | 59 |
| WW8 | KPGQSWWTDTGGIVHTFVVGDTKHPQSQDIYAKLKDLNVKMKEEGYVPHLDCVLWDISDDEKEDALCGHS EKLAIACALINTPPGTPIRIVKNLRVCDDCHKAIALISKIEGRNIICRDASRFHNYKDGKCSCGDYW | 60 |
| WW9 | KPGQSWWTDTGGIVHTFVVGDTKHPQSQDIYAKLKDLYVKMKEEGHVPHLDCVLWDISDDEKEDALCGHS EKLAIACALINTPPGTPIRIVKNLRVCDDCHKAIALISKIEGRNIICRDASRFHNYKDGKCSCGDYW | 61 |
| WW10 | KPGQSWWTDTGGIVHTFVVGDTKHPQSQDIYAKLKDLYVKMKEEGYVPHLDCVLWPISDDEKEDALCGHS EKLAIACALINTPPGTPIRIVKNLRVCDDCHKAIALISKIEGRNIICRDASRFHNYKDGKCSCGDYW | 62 |
| WW11 | KPGQSWWTDTGGIVHTFVVGDTKHPQSQDIYAKLKDLYVKMKEEGYVPHLDCVLWDISDDEKEDALCGHS EKLAIACALINTPPGTPIRVTKNLRVCDDCHKAIALISKIEGRNIICRDASRFHNYKDGKCSCGDYW | 63 |
| WW12 | KPGQSWWTDTGGIVHTFVVGDTKHPQSQDIYAKLKDLYVKMKEEGYVPHLDCVLWDISDDEKEDALCGHS EKLAIACALINTPPGTPIRIVKNLRMCDDCHKAIALISKIEGRNIICRDASRFHNYKDGKCSCGDYW | 64 |
| WW13 | KPGQSWWTDTGGIVHTFVVGDTKHPQSQDIYAKLKDLYVKMKEEGYVPHLDCVLWDISDDEKEDALCGHS EKLAIACALINTPPGTPIRIVKNLRVCDDCHKAIALISKIEKRNIICRDASRFHNYKDGKCSCGDYW | 65 |
| WW14 | KPGQSWWTDTGGIVHTFVVGDTKHPQSQDIYAKLKDLYVKMKEEGYVPHLDCVLWDISDDEKEDALCGHS EKLAIACALINTPPGTPIRIVKNLRVCDDCHKAIALISKIEGRNIICRDDCRFHNYKDGKCSCGDYW | 66 |
| WW15 | KPGQSWWTDTGGIVHTFVVGDTKHPQSQDIYAKLKDLYVKMKEEGYVPHLDCVLWDISDDEKEDALCGHS EKLAIACALINTPPGTPIRIVKNLRVCDDCHKAIALISKIEGRNIICRDASCVHNYKDGKCSCGDYW | 67 |

**[Table 4-3]**

| | Sequence | SEQ ID NO: |
|---|---|---|
| KP1 | KPASALIEVNKKVHEFVVGNNQSEEISAMLKSLNSRMKEEGHVPNLDLVLKPVSDEEKEAALCEHAEKLA IAFGLLNTPQGQTLRVTKNLRMCNDCHNASKIISKIERREIILRDDCCIHHFKDGLCSCGDMF | 3 |
| KP2 | KPAVAGIEVDRHVHTFVVGEERGDISCKVRSMNTRLKEEGGHVPHTELVLKPLSEKEKEDELCGHAEKLA LAYGLLNTPDGTPLLVTKNLRMCRDCHSSTEIMSRIEKREIVVRDAHRVHRFVDGSCSCGGRP | 68 |
| KP3 | KPAVATIEVNKQVHRFLVGERREDIALKVESTNARLKQEGGHVPHTKLVLKPLCEKEKEDALCGHAEKLA LAFGLLNTPSGTPLVVTKNLRMCSDCHSSTEIMSRLEQRDIVVRDGYRVHRFAAGCCSCKGRP | 69 |
| KP4 | KPAVATIEVNKQVHRFLVGERREDIALKVESTNARLKQEGGHVPHTKLVLKPLCEKEKEDVLCGHAEKLA LAFGLLNTPSGTPLVVTKNLRMCSDCHSSTEIMSRLEQRDIVVRDGYRVHRFANGCCSCKGRP | 70 |
| KP5 | KPGSALIEVNKKVHEFVVGNNQSEEISAMLKSLNSRMKEEGHVPNLDLVLKPVSDEEKEAALCEHAEKLA IAFGLLNTPQGQTLRVTKNLRMCNDCHNASKIISKIERREIILRDDCCIHHFKDGLCSCGDMF | 71 |
| KP6 | KPAQSWWTDTGGIVHTFVVGDTKHPQSEEISAMLKSLNSRMKEEGHVPNLDLVLKPVSDEEKEAALCEHA EKLAIAFGLLNTPQGQTLRVTKNLRMCNDCHNASKIISKIERREIILRDDCCIHHFKDGLCSCGDYW | 72 |
| KP7 | KPASSLIEVNKKVHEFVVGNNQSEEISAMLKSLNSRMKEEGHVPNLDLVLKPVSDEEKEAALCEHAEKLA IAFGLLNTPQGQTLRVTKNLRMCNDCHNASKIISKIERREIILRDDCCIHHFKDGLCSCGDMF | 73 |
| KP8 | KPGQAWWTDTGGIVHTFVVGDTKHPQSEEISAMLKSLNSRMKEEGHVPNLDLVLKPVSDEEKEAALCEHA EKLAIAFGLLNTPQGQTLRVTKNLRMCNDCHNASKIISKIERREIILRDDCCIHHFKDGLCSCGDYW | 74 |
| KP9 | KPASAWIEVNKKVHEFVVGNNQSEEISAMLKSLNSRMKEEGHVPNLDLVLKPVSDEEKEAALCEHAEKLA IAFGLLNTPQGQTLRVTKNLRMCNDCHNASKIISKIERREIILRDDCCIHHFKDGLCSCGDMF | 75 |
| KP10 | KPGQSTWTDTGGIVHTFVVGDTKHPQSEEISAMLKSLNSRMKEEGHVPNLDLVLKPVSDEEKEAALCEHA EKLAIAFGLLNTPQGQTLRVTKNLRMCNDCHNASKIISKIERREIILRDDCCIHHFKDGLCSCGDYW | 76 |
| KP11 | KPGQSWTDTGGIVHTFVVGDTKHPQSEEISAMLKSLNSRMKEEGHVPNLDLVLKPVSDEEKEAALCEHAE KLAIAFGLLNTPQGQTLRVTKNLRMCNDCHNASKIISKIERREIILRDDCCIHHFKDGLCSCGDYW | 77 |
| KP12 | KPGQSWWTDTGGIVHTFVVGDTKHPQSEEISAMLKSLSSRMKEEGHVPNLDLVLKPVSDEEKEAALCEHA EKLAIAFGLLNTPQGQTLRVTKNLRMCNDCHNASKIISKIERREIILRDDCCIHHFKDGLCSCGDYW | 78 |
| KP13 | KPASALIEVNKKVHEFVVGNNQSEEISAMLKSLNSRMKEEGYVPNLDLVLKPVSDEEKEAALCEHAEKLA IAFGLLNTPQGQTLRVTKNLRMCNDCHNASKIISKIERREIILRDDCCIHHFKDGLCSCGDMF | 79 |
| KP14 | KPGQSWWTDTGGIVHTFVVGDTKHPQSEEISAMLKSLNSRMKEEGYVPNLDLVLKPVSDEEKEAALCEHA EKLAIAFGLLNTPQGQTLRVTKNLRMCNDCHNASKIISKIERREIILRDDCCIHHFKDGLCSCGDYW | 80 |
| KP15 | KPGQSWWTDTGGIVHTFVVGDTKHPQSEEISAMLKSLNSRMKEEGHVPNLDLVLKDVSDEEKEAALCEHA EKLAIAFGLLNTPQGQTLRVTKNLRMCNDCHNASKIISKIERREIILRDDCCIHHFKDGLCSCGDYW | 81 |
| KP16 | KPGQSWWTDTGGIVHTFVVGDTKHPQSEEISAMLKSLNSRMKEEGHVPNLDLVLKPVSDEEKEAALCEHA EKLAIAFGLLNTPQGQTLRIVKNLRMCNDCHNASKIISKIERREIILRDDCCIHHFKDGLCSCGDYW | 82 |
| KP17 | KPASALIEVNKKVHEFVVGNNQSEEISAMLKSLNSRMKEEGHVPNLDLVLKPVSDEEKEAALCEHAEKLA IAFGLLNTPQGQTLRVTKNLRMCNDCHNASKIISKIEGREIILRDDCCIHHFKDGLCSCGDMF | 83 |
| KP18 | KPGQSWWTDTGGIVHTFVVGDTKHPQSEEISAMLKSLNSRMKEEGHVPNLDLVLKPVSDEEKEAALCEHA EKLAIAFGLLNTPQGQTLRVTKNLRMCNDCHNASKIISKIEGREIILRDDCCIHHFKDGLCSCGDYW | 84 |
| KP19 | KPASALIEVNKKVHEFVVGNNQSEEISAMLKSLNSRMKEEGHVPNLDLVLKPVSDEEKEAALCEHAEKLA IAFGLLNTPQGQTLRVTKNLRMCNDCHNASKIISKIERREIILRDASCIHHFKDGLCSCGDMF | 85 |
| KP20 | KPGQSWWTDTGGIVHTFVVGDTKHPQSEEISAMLKSLNSRMKEEGHVPNLDLVLKPVSDEEKEAALCEHA EKLAIAFGLLNTPQGQTLRVTKNLRMCNDCHNASKIISKIERREIILRDASCIHHFKDGLCSCGDYW | 86 |
| KP21 | KPGQSWWTDTGGIVHTFVVGDTKHPQSEEISAMLKSLNSRMKEEGHVPNLDLVLKPVSDEEKEAALCEHA EKLAIAFGLLNTPQGQTLRVTKNLRMCNDCHNASKIISKIERREIILRDDCCIHHFKDGLCSCGDMF | 87 |
| KP22 | KPGQSWWTDTGGIVHTFVVGDTKHPQSQDIYAKLKSLNSRMKEEGHVPNLDLVLKPVSDEEKEAALCEHA EKLAIAFGLLNTPQGQTLRVTKNLRMCNDCHNASKIISKIERREIILRDDCCIHHFKDGLCSCGDMF | 88 |
| KP23 | KPGQSWWTDTGGIVHTFVVGDTKHPQSQDIYAKLKSLNSRMKEEGHVPNLDLVLKPVSDEEKEAALCEHA EKLAIAFGLLNTPQGQTLRVTKNLRMCNDCHNASKIISKIERREIILRDDCCIHHFKDGLCSCGDYW | 89 |
| chim KPla | KPGQSWWTDTGGIVHTFVVGDTKHPQSEEISAMLKSLNSRMKEEGHVPNLDLVLKPVSDEEKEAALCEHA EKLAIAFGLLNTPQGQTLRVTKNLRMCNDCHNASKIISKIERREIILRDDCCIHHFKDGLCSCGDYW | 90 |

**[Table 4-4]**

| | Sequence | SEQ ID NO : |
|---|---|---|
| MTS-P-PG1_ND 2 | MALLRAAVSELRRRGRGALTPLPALSSLLSSLSPRSPASTRPEPNNPHADRRHVIALRRCPPLPASAVLAP ELLHARGLLPRHWSHASPLSTSSSSSRPADKAQLTWVDKWIPEAARPYSHGSGGSGGGLHMGSGGLSSGDY KDHDGDYKDHDIDYKDDDDKGSGGSGGSGNSVVTYNTLIDGLCKSGKIEEALKLFKEMEEKGITPSVVTYN TLIDGLCKSGKIEEALKLFKEMEEKGITPSVVTYNTLIDGLCKSGKIEEALKLFKEMEEKGITPSVVTYTT LIDGLCKAGKVDEALELFKEMRSKGVKPNVVTYTTLIDGLCKAGKVDEALELFDEMKERGIKPDVVTYNTL IDGLCKSGKIEEALKLFKEMEEKGITPSVVTYNTLIDGLCKAGRLDEAEELLEEMEEKGIKPDVVTYTTLI DGLCKAGKVDEALELFKEMRSKGVKPNVVTYNTLIDGLCKSGKIEEALKLFKEMEEKGITPSVVTYTTLID GLCKAGKVDEALELFDEMKERGIKPDVVSWNAMIAAYAQHGHGKEALQLFQQMQQEGVKPSEVTFTSILSA CSHAGLVDEGHHYFESMSPDYGITPRVEHYGCMVDLLGRAGRLDEAEDLIKSMPFQPNVVVWGTLLGACRV HGDVERGERAAERILELDPESAAPYVLLSNIYAAAGRWDEAAKVRKLMKERGVKKEPGCSWIEVNNKVHEF VAGDKSHPQTKEIYAELERLSKQMKEAGYVPDTKFVLHDVEEEEKEQLLCYHSEKLAIAFGLISTPPGTPL RIIKNLRVCGDCHTATKFISKIVGREIVVRDANRFHHFKDGVCSCGDYW | 93 |
| MTS-P-WW1_ND 2 | MALLRAAVSELRRRGRGALTPLPALSSLLSSLSPRSPASTRPEPNNPHADRRHVIALRRCPPLPASAVLAP ELLHARGLLPRHWSHASPLSTSSSSSRPADKAQLTWVDKWIPEAARPYSHGSGGSGGGLHMGSGGLSSGDY KDHDGDYKDHDIDYKDDDDKGSGGSGGSGNSVVTYNTLIDGLCKSGKIEEALKLFKEMEEKGITPSVVTYN TLIDGLCKSGKIEEALKLFKEMEEKGITPSVVTYNTLIDGLCKSGKIEEALKLFKEMEEKGITPSVVTYTT LIDGLCKAGKVDEALELFKEMRSKGVKPNVVTYTTLIDGLCKAGKVDEALELFDEMKERGIKPDVVTYNTL IDGLCKSGKIEEALKLFKEMEEKGITPSVVTYNTLIDGLCKAGRLDEAEELLEEMEEKGIKPDVVTYTTLI DGLCKAGKVDEALELFKEMRSKGVKPNVVTYNTLIDGLCKSGKIEEALKLFKEMEEKGITPSVVTYTTLID GLCKAGKVDEALELFDEMKERGIKPDVVTWNALIAGYARQGESDLVFHLLERMRQEGIQPSGVTFTSVLTV CSHAGLVDEGQKYFDAMSEDYGITPRIEHYGCMVDLLGRAGQMDEAVAMVEKMPFQPNLVTWGTLLGACRK WNNVEIGRHAFECAVRLDEKSAAAYVLMSNIYADAHMWEERDKIQAMRKNARAWKKPGQSWWTDTGGIVHT FVVGDTKHPQSQDIYAKLKDLYVKMKEEGYVPHLDCVLWDISDDEKEDALCGHSEKLAIACALINTPPGTP IRIVKNLRVCDDCHKAIALISKIEGRNIICRDASRFHNYKDGKCSCGDYW | 94 |
| MTS-P-PG1_ND 5 | MALLRAAVSELRRRGRGALTPLPALSSLLSSLSPRSPASTRPEPNNPHADRRHVIALRRCPPLPASAVLAP ELLHARGLLPRHWSHASPLSTSSSSSRPADKAQLTWVDKWIPEAARPYSHGSGGSGGGLHMGSGGLSSGDY KDHDGDYKDHDIDYKDDDDKGSGGSGGSGNSVVTYTTLIDGLCKAGKVDEALELFKEMRSKGVKPNVVTYN TLIDGLCKAGRLDEAEELLEEMEEKGIKPDVVTYTTLIDGLCKAGKVDEALELFDEMKERGIKPDVVTYTT LIDGLCKAGKVDEALELFKEMRSKGVKPNVVTYNTLIDGLCKSGKIEEALKLFKEMEEKGITPSVVTYTTL IDGLCKAGKVDEALELFKEMRSKGVKPNVVTYNTLIDGLCKAGRLDEAEELLEEMEEKGIKPDVVTYNTLI DGLCKSGKIEEALKLFKEMEEKGITPSVVTYTTLIDGLCKAGKVDEALELFKEMRSKGVKPNVVTYTTLID GLCKAGKVDEALELFKEMRSKGVKPNVVSWTAMIAAYAQHGHGKEALQLFQQMQQEGVKPNEVTFTSILSA CSHAGLVDEGHHYFESMSPDYGITPRVEHYGCMVDLLGRAGRLDEAEDLIKSMPFQPNVVVWGTLLGACRV HGDVERGERAAERILELDPESAAPYVLLSNIYAAAGRWDEAAKVRKLMKERGVKKEPGCSWIEVNNKVHEF VAGDKSHPQTKEIYAELERLSKQMKEAGYVPDTKFVLHDVEEEEKEQLLCYHSEKLAIAFGLISTPPGTPL RIIKNLRVCGDCHTATKFISKIVGREIVVRDANRFHHFKDGVCSCGDYW | 95 |
| MTS-P-WW1_ND 5 | MALLRAAVSELRRRGRGALTPLPALSSLLSSLSPRSPASTRPEPNNPHADRRHVIALRRCPPLPASAVLAP ELLHARGLLPRHWSHASPLSTSSSSSRPADKAQLTWVDKWIPEAARPYSHGSGGSGGGLHMGSGGLSSGDY KDHDGDYKDHDIDYKDDDDKGSGGSGGSGNSVVTYTTLIDGLCKAGKVDEALELFKEMRSKGVKPNVVTYN TLIDGLCKAGRLDEAEELLEEMEEKGIKPDVVTYTTLIDGLCKAGKVDEALELFDEMKERGIKPDVVTYTT LIDGLCKAGKVDEALELFKEMRSKGVKPNVVTYNTLIDGLCKSGKIEEALKLFKEMEEKGITPSVVTYTTL IDGLCKAGKVDEALELFKEMRSKGVKPNVVTYNTLIDGLCKAGRLDEAEELLEEMEEKGIKPDVVTYNTLI DGLCKSGKIEEALKLFKEMEEKGITPSVVTYTTLIDGLCKAGKVDEALELFKEMRSKGVKPNVVTYTTLID GLCKAGKVDEALELFKEMRSKGVKPNVVTWTALIAGYARQGESDLVFHLLERMRQEGIQPNGVTFTSVLTV CSHAGLVDEGQKYFDAMSEDYGITPRIEHYGCMVDLLGRAGQMDEAVAMVEKMPFQPNLVTWGTLLGACRK WNNVEIGRHAFECAVRLDEKSAAAYVLMSNIYADAHMWEERDKIQAMRKNARAWKKPGQSWWTDTGGIVHT FVVGDTKHPQSQDIYAKLKDLYVKMKEEGYVPHLDCVLWDISDDEKEDALCGHSEKLAIACALINTPPGTP IRIVKNLRVCDDCHKAIALISKIEGRNIICRDASRFHNYKDGKCSCGDYW | 96 |

## Claims

1. A method for editing a target RNA, the method comprising
applying to the target RNA an artificial DYW protein containing a DYW domain consisting of any one of the polypeptides a, b, c, and bc mentioned below:
a. a polypeptide having xₐ₁PGxₐ₂SWIExₐ₃-xₐ₁₆HP ... HxₐₐE ... Cxₐ₁₇xₐ₁₈CH ... DYW, having a sequence identity of at least 40% to the sequence of SEQ ID NO: 1, and having a C-to-U/U-to-C editing activity,
b. a polypeptide having x_{b1}PGx_{b2}SWWTDx_{b3}-x_{b16}HP ... Hx_{bb}E ... Cx_{b17}x_{b18}CH ... DYW, having a sequence identity of at least 40% to the sequence of SEQ ID NO: 2, and having a C-to-U/U-to-C editing activity,
c. a polypeptide having KPAx_{c1}Ax_{c2}IEx_{c3} ... Hx_{cc}E ... Cx_{c4}x_{c5}CH ... x_{c6}x_{c7}x_{c8}, having a sequence identity of at least 40% to the sequence of SEQ ID NO: 3, and having a C-to-U/U-to-C editing activity, and
bc. a polypeptide having x_{b1}PGx_{b2}SWWTDx_{b3}-x_{b16}HP ... Hx_{cc}E ... Cx_{c4}x_{c5}CH ... Dx_{bc1}x_{bc2}, having a sequence identity of at least 40% to the sequence of SEQ ID NO: 90, and having a C-to-U/U-to-C editing activity
(in the sequences, x represents an arbitrary amino acid, and ... represents an arbitrary polypeptide fragment).

2. A DYW domain consisting of any one of the polypeptides a, b, c, and bc mentioned below:
a. a polypeptide having xₐ₁PGxₐ₂SWIExₐ₃-xₐ₁₆HP ... HxₐₐE ... Cxₐ₁₇xₐ₁₈CH ... DYW, having a sequence identity of at least 40% to the sequence of SEQ ID NO: 1, and having a C-to-U/U-to-C editing activity,
b. a polypeptide having x_{b1}PGx_{b2}SWWTDx_{b3}-x_{b16}HP ... Hx_{bb}E ... Cx_{b17}x_{b18}CH ... DYW, having a sequence identity of at least 40% to the sequence of SEQ ID NO: 2, and having a C-to-U/U-to-C editing activity,
c. a polypeptide having KPAx_{c1}Ax_{c2}IEx_{c3} ... Hx_{cc}E ... Cx_{c4}x_{c5}CH ... x_{c6}x_{c7}x_{c8}, having a sequence identity of at least 40% to the sequence of SEQ ID NO: 3, and having a C-to-U/U-to-C editing activity, and
bc. a polypeptide having x_{b1}PGx_{b2}SWWTDx_{b3}-x_{b16}HP ... Hx_{cc}E ... Cx_{c4}x_{c5}CH ... Dx_{bc1}x_{bc2}, having a sequence identity of at least 40% to the sequence of SEQ ID NO: 90, and having a C-to-U/U-to-C editing activity.

3. A DYW protein containing
an RNA-binding domain that contains at least one PPR motif and can sequence-specifically bind to a target RNA, and
the DYW domain according to claim 2.

4. The DYW protein according to claim 3, wherein the RNA-binding domain is of the PLS type.

5. A method for editing a target RNA, the method comprising:
applying to the target RNA a DYW domain consisting of the polypeptide c or be mentioned below to convert an editing target U to C:
c. a polypeptide having KPAx_{c1}Ax_{c2}IEx_{c3} ... Hx_{cc}E ... Cx_{c4}x_{c5}CH ... x_{c6}x_{c7}x_{c8}, having a sequence identity of at least 40% to the sequence of SEQ ID NO: 3, and having a C-to-U/U-to-C editing activity, and
be. a polypeptide having x_{b1}PGx_{b2}SWWTDx_{b3}-x_{b16}HP ... Hx_{cc}E ... Cx_{c4}x_{c5}CH ... Dx_{bc1}x_{bc2}, having a sequence identity of at least 40% to the sequence of SEQ ID NO: 90, and having a C-to-U/U-to-C editing activity.

6. The method according to claim 5, wherein the DYW domain is fused to an RNA-binding domain that contains at least one PPR motif, and can sequence-specifically bind to a target RNA according to the rules of the PPR codes.

7. A composition for editing RNA in an eukaryotic cell, the composition containing the DYW domain according to claim 2.

8. A nucleic acid encoding the DYW domain according to claim 2, or the DYW protein according to claim 3 or 4.

9. A vector containing the nucleic acid according to claim 8.

10. A cell (except for human individual) containing the vector according to claim 9.
